# EUROPEAN PATENT SPECIFICATION

(11) **EP 2 948 143 B1**
(45) Date of publication and mention of the grant of the patent: **05.05.2021**
(21) Application number: 14743158.9
(22) Date of filing: 24.01.2014
(51) Int. Cl.: A61K 31/4245, A61K 31/352, A61K 31/4025, A61P 25/00, A61P 25/28, A61P 25/24, A61P 25/20, C07D 413/14

(54) **USE OF PHENOXYPROPYLAMINE COMPOUNDS TO TREAT DEPRESSION**
VERWENDUNG VON PHENOXYPROPYLAMINVERBINDUNGEN ZUR BEHANDLUNG VON DEPRESSIONEN
UTILISATION DE COMPOSÉS DE PHÉNOXYPROPYLAMINE POUR TRAITER LA DÉPRESSION

(30) Priority: 24.01.2013 US 201361756208 P; 15.03.2013 US 201361799482 P; 15.03.2013 US 201361852149 P
(43) Date of publication of application: 02.12.2015
(73) Proprietor: Minerva Neurosciences, Inc., Waltham, MA 02451 (US)
(72) Inventor: PELLEGRINI, Lorenzo, Newtown, PA 18940 (US); KARABELAS, Argeris, Portsmouth, NH 03801 (US); LUTHRINGER, Remy, CH-1204 Geneva (CH)
(74) Representative: Cooley (UK) LLP
(86) International application number: PCT/US2014/013026
(87) International publication number: WO 2014/117003

(56) References cited:
- US-A- 4 207 343
- US-A1- 2004 138 227
- PAPP M ET AL: "P.2.b.008 Antidepressant-like activity of Wf-516, an antagonist of 5-HT1A receptors and inhibitor of 5-HT reuptake, in a chronic mild stress model of depression in rat", EUROPEAN NEUROPSYCHOPHARMACOLOGY, ELSEVIER SCIENCE PUBLISHERS BV, AMSTERDAM, NL, vol. 16, 1 January 2006 (2006-01-01), pages S305-S306, XP027969671, ISSN: 0924-977X [retrieved on 2006-01-01]
- EL MANSARI M ET AL: "In vivo electrophysiological assessment of the putative antidepressant Wf-516 in the rat raphe dorsalis, locus coeruleus and hippocampus", NAUNYN-SCHMIEDEBERG'S ARCHIVES OF PHARMACOLOGY, SPRINGER, BERLIN, DE, vol. 376, no. 5, 30 November 2007 (2007-11-30), pages 351-361, XP019584490, ISSN: 1432-1912
- TAKEAKI SAIJO ET AL: "Presynaptic Selectivity of a Ligand for Serotonin 1A Receptors Revealed by In Vivo PET Assays of Rat Brain", PLOS ONE, vol. 7, no. 8, 7 August 2012 (2012-08-07), page e42589, XP055297335, DOI: 10.1371/journal.pone.0042589
- NUTT DAVID ET AL: "Sleep disorders as core symptoms of depression.", DIALOGUES IN CLINICAL NEUROSCIENCE 2008, vol. 10, no. 3, 2008, pages 329-336, XP002761112, ISSN: 1294-8322
- SCHMITT J A J ET AL: "Serotonin and human cognitive performance.", CURRENT PHARMACEUTICAL DESIGN 2006, vol. 12, no. 20, 2006, pages 2473-2486, XP002761113, ISSN: 1381-6128
- SHANNON REAGAN-SHAW ET AL: "Dose translation from animal to human studies revisited", THE FASEB JOURNAL, vol. 22, no. 3, 1 March 2008 (2008-03-01), pages 659-661, XP055542714, US ISSN: 0892-6638, DOI: 10.1096/fj.07-9574LSF
- Anonymous: "Guidance for Industry - Estimating the Maximum Safe Starting Dose in Initial Clinical Trials for Therapeutics in Adult Healthy Volunteers", , 1 July 2005 (2005-07-01), pages FP-3,1-27, XP055294866, Retrieved from the Internet: URL:http://www.fda.gov/downloads/Drugs/... /Guidances/UCM078932.pdf [retrieved on 2016-08-10]

## Description

### FIELD OF THE INVENTION

The present invention in some embodiments relates to compositions and methods of treating depression in a patient.

### BACKGROUND OF THE INVENTION

Certain diseases of the central nervous system (e.g., depression, anxiety) are considered to be caused by disorders of noradrenaline (NA) and 5-hydroxytryptamine (5-HT, also known as serotonin), which are neurotransmitters. Accordingly, augmentation of 5-HT-related neurotransmission is considered to mainly influence depressive mood and anxiety, whereas augmentation of noradrenergic neurotransmission is considered to influence retardation in depressive patients. Pharmaceutical agents such as imipramine, desipramine and the like, which are most frequently used for the treatment of depression, are considered to act on depressive patients by improving neurotransmission of one or both of NA and 5-HT receptors.

The activity of 5-HT is considered to relate to a number of various types of psychiatric disorders. In addition, 5-HT has been considered to be responsible for various conditions (e.g., eating disorder, gastrointestinal injury, control of cardiovascular system, cognition, and sexual behavior). However, conventional antidepressants, such as imipramine, desipramine and the like, are defective in that they require 3-4 weeks or even longer time for the expression of an anti-depressive effect, which poses clinical problems.

A combined use of various pharmaceutical agents has been considered in an attempt to accelerate expression of effects of antidepressants or to increase their efficacy (Journal of Clinical Psychiatry, Vol. 57; Supplement 7; pp 25-31). Therein, a noticeably shortened time for clinical expression of the effect by concurrent use of a selective serotonin (5-HT) reuptake inhibitor (SSRI) and a 5-HT_{1A} antagonist, pindolol, has been reported (Journal of Clinical Psychopharmacology, Vol. 17, No. 6, pp. 446-450). It is known that the amount of 5-HT release in the brain does not increase much by SSRI alone, but when combined with a 5-HT_{1A} antagonist, the amount increases markedly (Neurochemical Research, Vol. 21, No. 5, 1996, pp. 557-562). Under such circumstances, the "5-HT enhancement hypothesis" was proposed with regard to the expression of the action of antidepressants by Blier and de Montigny (Trends in Pharmacological Sciences, 1994, vol. 15, pp. 220-226). The 5-HT enhancement hypothesis means that the effector mechanism of antidepressant rests in the enhancement of 5-HT release at a terminal. It is based on the understanding that the conventional antidepressants decrease the 5-HT release by single administration, but increase the 5-HT release and express an anti-depressive effect only when they are administered consecutively. From those mentioned above, it is expected that a drug that promotes 5-HT release in the brain from the first can be a rapid onset antidepressant. In other words, a compound concurrently having a serotonin reuptake inhibitory action and a 5-HT_{1A} antagonistic action is considered to be an antidepressant showing quick expression of an anti-depressive effect, namely, a rapid onset antidepressant.

There remains a need to identify medicaments and methods for use in the treatment of depression, and furthermore, compositions and methods of treatment which improve on the efficacy of existing therapies.

### SUMMARY OF THE INVENTION

In the disclosure encompassed herein, compounds of formula I have been shown to have properties useful to treat depression and/or one or more symptoms of depression. Disclosed herein therefore are methods and compositions for treating various aspects of depression. The present invention provides a composition for use in treating or improving at least one disorder or parameter of sleep in a human subject, the composition comprising the compound of formula (I) or a pharmaceutically acceptable salt, hydrate, or solvate thereof, 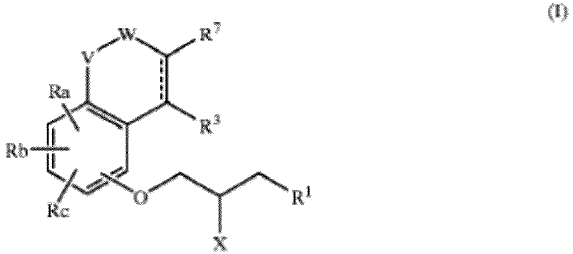 wherein each symbol in the formula means as follows: a bond represented by a solid line and a dotted line shows a double bond or a single bond;
X is a hydrogen atom, a hydroxy group, a C₁ -C₈ alkoxy group, an acyloxy group or an oxo group;
R¹ is a group of the following formula 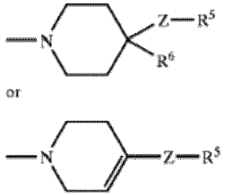
wherein R⁵ is optionally substituted aryl group or optionally substituted aromatic heterocyclic group,
Z is void or --CH₂ --, and
R⁶ is hydrogen atom, hydroxy group, acetamido group, carboxyl group, alkoxycarbonyl group, cyano group or C₁ -C₈ alkoxy group;
R³ is a hydrogen atom, a C₁ -C₁₈ alkyl group or a halogen atom;
V is --O--;
W is void;
R⁷ is a C₁ -C₄ hydroxyalkyl group, an acyl group, an optionally substituted saturated or unsaturated heterocyclic group, an optionally substituted fused heterocyclic group, a C₁ -C₄ alkylsulfonyl group or the formula --Q--R⁹
wherein
Q is --C(=O)--, --C(=S)--, --CH₂ -- or --S(=O)₂--, and
R⁹ is a group of the following formula 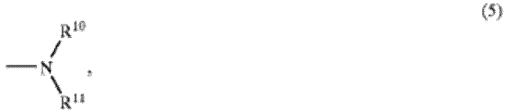 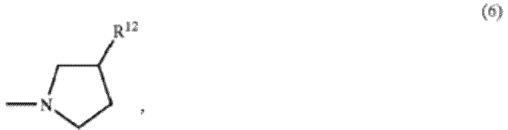 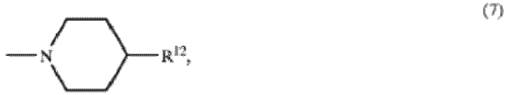 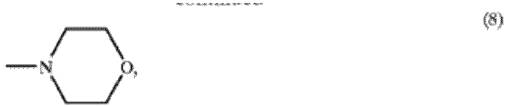 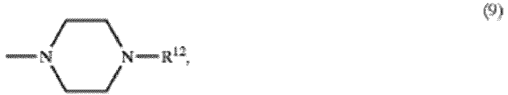 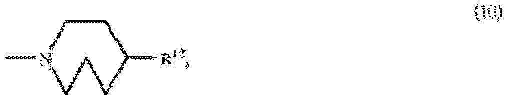 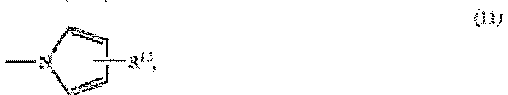 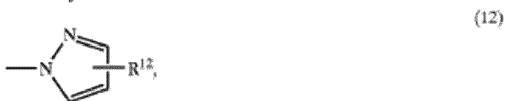 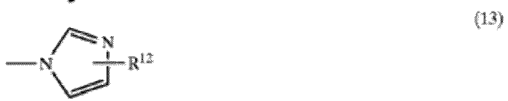 or --NH--NH--R¹⁵
wherein R¹⁰ and R¹¹ are each independently hydrogen atom, C₁ -C₁₈ alkyl group, optionally substituted aryl group, optionally substituted aralkyl group or alkoxy group, R¹² is hydrogen atom, optionally substituted aryl group, C₁ -C₁₈ alkyl group, C₁ -C₈ alkoxy group or acyl group, and R¹⁵ is hydrogen atom, phenyl group, C₁ -C₄ alkyl group, C₁ -C₂ halogenated alkyl group, halogen atom, C₂ -C₄ alkenyl group, C₁ -C₄ hydroxyalkyl group, alkoxyalkyl group, alkyloxycarbonyl group, optionally substituted amino group, acetamido group, carboxyl group, acyl group, optionally substituted alkyloxy group, alkylthio group or cyano group;
Ra, Rb and Rc are each independently a hydrogen atom, a C₁ -C₁₈ alkyl group, a hydroxy group, a Ci -C₈ alkoxy group, a halogen atom, an acyl group, a nitro group or an amino group;
an optically active compound thereof, a pharmaceutically acceptable salt thereof or a hydrate thereof;
wherein the compound of formula (I) is present in the composition between 0.01 mg/kg and 0.2 mg/kg of the subject's weight, and the subject suffers from depression.

In an embodiment, a composition is provided for treating or diminishing at least one symptom of depression in a human subject comprising administering to a subject in need thereof a therapeutically effective amount of a composition comprising the compound of formula I or a pharmaceutically acceptable salt, hydrate, or solvate thereof.

In an embodiment, a composition is provided for treating or diminishing at least one symptom of depression in a human subject comprising administering to a subject in need thereof a therapeutically effective amount of a composition comprising the compound of formula II or a pharmaceutically acceptable salt, hydrate, or solvate thereof.

Also disclosed is a method of treating depression comprising the step of administering a low dose of a compound of formula I, or a pharmaceutically acceptable salt, hydrate, or solvate thereof, to a subject in need thereof.

Also disclosed is a method of treating depression comprising the step of administering a low dose of a compound of formula II, or a pharmaceutically acceptable salt, hydrate, or solvate thereof, to a subject in need thereof.

### BRIEF DESCRIPTION OF THE DRAWINGS

The foregoing summary, as well as the following detailed description of the invention, will be better understood when read in conjunction with the appended drawings.
Figure 1 illustrates the effect of imipramine in chronic mild stress. Shown are the effects of chronic treatment with vehicle (1 mL/kg, IP) and imipramine (10 mg/kg, IP) on the consumption of 1% sucrose solution in controls (open symbols) and in animals exposed to chronic mild stress (closed symbols). Treatment commenced following 2 weeks of initial stress. Values are means +/- SEM.
Figure 2 illustrates the effect of SON-117 in chronic mild stress. Shown are the effects of chronic treatment with vehicle (1 mL/kg, IP) and SON-117 (0.001, 0.01, 0.1, and 1 mg/kg, IP) on the consumption of 1% sucrose solution in controls (open symbols) and in animals exposed to chronic mild stress (closed symbols). Treatment commenced following 2 weeks of stress. Values are means +/- SEM.
Figure 3 illustrates the effect of imipramine and SON-117 in the novel object recognition test. Shown are the effects of chronic treatment with vehicle (1 mL/kg, IP) imipramine (10 mg/kg, IP), and SON-117 (0.001, 0.01, 0.1, and 1 mg/kg, IP) on the behavior of control (open symbols) and stressed (closed symbols) animals in the novel object recognition test. The test was carried out one week after withdrawal from the treatments. Values are means +/- SEM.
Figure 4 illustrates the effect of citalopram on norepinephrin release in the rat prefrontal cortex (n = 8).
Figure 5 illustrates the effect of citalopram on DOPAC release in the rat prefrontal cortex (n = 8).
Figure 6 illustrates the effect of citalopram on dopamine release in the rat prefrontal cortex (n = 8).
Figure 7 illustrates the effect of citalopram on 5-HIAA release in the rat prefrontal cortex (n = 8).
Figure 8 illustrates the effect of citalopram on HVA release in the rat prefrontal cortex (n = 8).
Figure 9 illustrates the effect of citalopram on 5-HT release in the rat prefrontal cortex (n = 8).
Figure 10 illustrates the effect of SON-117 (0.1 mg/kg) on NE release in the rat prefrontal cortex (n = 8).
Figure 11 illustrates the effect of SON-117 (0.1 mg/kg) on DOPAC release in the rat prefrontal cortex (n = 8).
Figure 12 illustrates the effect of SON-117 (0.1 mg/kg) on dopamine release in the rat prefrontal cortex (n = 8).
Figure 13 illustrates the effect of SON-117 (0.1 mg/kg) on 5-HIAA release in the rat prefrontal cortex (n = 8).
Figure 14 illustrates the effect of SON-117 (0.1 mg/kg) on HVA release in the rat prefrontal cortex (n = 8).
Figure 15 illustrates the effect of SON-117 (0.1 mg/kg) on 5-HT release in the rat prefrontal cortex (n = 8).
Figure 16 illustrates the effect of SON-117 (0.3 mg/kg) on NE release in the rat prefrontal cortex (n = 8).
Figure 17 illustrates the effect of SON-117 (0.3 mg/kg) on DOPAC release in the rat prefrontal cortex (n = 8).
Figure 18 illustrates the effect of SON-117 (0.3 mg/kg) on dopamine release in the rat prefrontal cortex (n = 8).
Figure 19 illustrates the effect of SON-117 (0.3 mg/kg) on 5-HIAA release in the rat prefrontal cortex (n = 8).
Figure 20 illustrates the effect of SON-117 (0.3 mg/kg) on HVA release in the rat prefrontal cortex (n = 8).
Figure 21 illustrates the effect of SON-117 (0.3 mg/kg) on 5-HT release in the rat prefrontal cortex (n = 8).
Figure 22 illustrates the effect of SON-117 (3 mg/kg) on NE release in the rat prefrontal cortex (n = 8).
Figure 23 illustrates the effect of SON-117 (3 mg/kg) on DOPAC release in the rat prefrontal cortex (n = 8).
Figure 24 illustrates the effect of SON-117 (3 mg/kg) on dopamine release in the rat prefrontal cortex (n = 8).
Figure 25 illustrates the effect of SON-117 (3 mg/kg) on 5-HIAA release in the rat prefrontal cortex (n = 8).
Figure 26 illustrates the effect of SON-117 (3 mg/kg) on HVA release in the rat prefrontal cortex (n = 8).
Figure 27 illustrates the effect of SON-117 (3 mg/kg) on 5-HT release in the rat prefrontal cortex (n = 8).
Figure 28 illustrates the effect of SON-117 versus citalopram on NE release in rat prefrontal cortex. Values of mean NE levels (+/- SEM) measured in each experimental group (n = 8).
Figure 29 illustrates the effect of SON-117 versus citalopram on DOPAC release in rat prefrontal cortex. Values of mean DOPAC levels (+/- SEM) measured in each experimental group (n = 8).
Figure 30 illustrates the effect of SON-117 versus citalopram on dopamine release in rat prefrontal cortex. Values of mean dopamine levels (+/- SEM) measured in each experimental group (n = 8).
Figure 31 illustrates the effect of SON-117 versus citalopram on 5-HIAA release in rat prefrontal cortex. Values of mean 5-HIAA levels (+/- SEM) measured in each experimental group (n = 8).
Figure 32 illustrates the effect of SON-117 versus citalopram on HVA release in rat prefrontal cortex. Values of mean HVA levels (+/- SEM) measured in each experimental group (n = 8).
Figure 33 illustrates the effect of SON-117 versus citalopram on 5-HT release in rat prefrontal cortex. Values of mean 5-HT levels (+/- SEM) measured in each experimental group (n = 8).
Figure 34 illustrates pharmacokinetic data for SON-117 at day 1.
Figure 35 illustrates pharmacokinetic data for SON-117 at day 14.
Figure 36 illustrates pharmacokinetic data for M1 at day 1.
Figure 37 illustrates pharmacokinetic data for M1 at day 14.

### DETAILED DESCRIPTION OF THE INVENTION

In the disclosure encompassed herein, compounds of formula I have been shown to have properties useful to treat depression and/or one or more symptoms of depression. Encompassed herein therefore are methods and compositions for treating various aspects of depression.

It is an object of the present disclosure to provide methods of treating or diminishing at least one symptom of depression in a human subject comprising administering to a subject in need thereof a therapeutically effective amount of a composition comprising the compound of formula (I) or a pharmaceutically acceptable salt, hydrate, or solvate thereof. It is also an object of the present disclosure to provide compositions comprising the compound of formula (I) or a pharmaceutically acceptable salt, hydrate, or solvate thereof for treating or diminishing at least one symptom of depression in a human subject comprising administering to a subject in need thereof. It is a further object of the present disclosure to provide compositions and methods for treating or improving at least one disorder or parameter of sleep in a subject comprising a human subject comprising administering to a subject in need thereof a therapeutically effective amount of a composition comprising the compound of formula (I) or a pharmaceutically acceptable salt, hydrate, or solvate thereof.

U.S. Patent 6,720,320 discloses phenoxypropylamine compounds and derivatives having selective affinity for and antagonistic activity against the 5-HT_{1A} receptor, as well as 5-HT reuptake inhibitory activity. Certain compounds disclosed therein also demonstrate fast-acting antidepressant activity. Such compounds may be useful for treatment of diseases that can be therapeutically and/or preventively treated by antagonistic activity against the 5-HT_{1A} receptor and/or inhibition of 5-HT reuptake. However, certain properties and characteristics of specific derivatives were not disclosed in U.S. Patent 6,720,320. Notably, U.S. Patent 6,720,320 states that the general daily dose of the described phenoxypropylamine compounds in the case of oral administration is 0.5-10 mg/kg, preferably 1-5 mg/kg. It has been unexpectedly observed in the present disclosure that effective treatment of selected disorders can occur with oral dosing that approaches 0.001 mg/kg.

For purposes of the disclosure encompassed herein, the term "mood disorders" is to be understood as encompassing those conditions defined as mood disorders in the Diagnostic and Statistical Manual of Mental Disorders, Fifth Edition, 2000 ("DSM-IV-TR"). More specifically, the mood disorders to be treated in accordance with the disclosure encompassed herein include those associated with the occurrence of more than one depressive symptom, such as depressed mood, loss of interest or pleasure, loss of appetite, sleep disturbance, psychomotor changes, fatigue, a sense of worthlessness, impaired concentration or thoughts of death, the symptoms typically being exhibited over a prolonged period of time. In an embodiment, compounds of formula I are used to treat those conditions defined in DSM-IV-TR as being associated with a Major Depressive Episode, i.e., a period of at least two weeks in which the individual to be treated exhibits either depressed mood, or the loss of interest or pleasure, associated with at least four additional depressive symptoms from the list cited above. Examples of such conditions include Major Depressive Disorder, Bipolar I Disorder, and Bipolar II Disorder. In another embodiment, the condition to be treated is characterized by depressive symptoms that do not necessarily rise to the level of a Major Depressive Episode, such as Dysthymic Disorder, Depressive Disorder Not Otherwise Specified, Cyclothymic Disorder, Bipolar Disorder Not Otherwise Specified, Mood Disorder Due to a General Medical Condition, and Substance-Induced Mood Disorder. In another embodiment, compounds of formula I can be used to treat isolated Major Depressive Episodes that are not characterized by DSM-IV-TR as a defined disorder, for example, those associated with post-partum depression as well as depressive symptoms associated with mental conditions that are not formally classified as mood disorders, for example, Schizoaffective Disorder or Seasonal Affective Disorder. In yet another embodiment, compounds of formula I can be used to treat an individual experiencing two or more depressive symptoms, which condition is not characterized according to DSM-IV-TR as a Major Depressive Episode. Each of the embodiments noted above shall be understood to be encompassed within the term "depression-related mood disorders".

### Compounds and Methods for Treating Depression

In an embodiment, a composition is provided for use in treating or improving at least one disorder or parameter of sleep in a human subject comprising administering to a subject in need thereof a therapeutically effective amount of a composition comprising the compound of formula (I) or a pharmaceutically acceptable salt, hydrate, or solvate thereof, 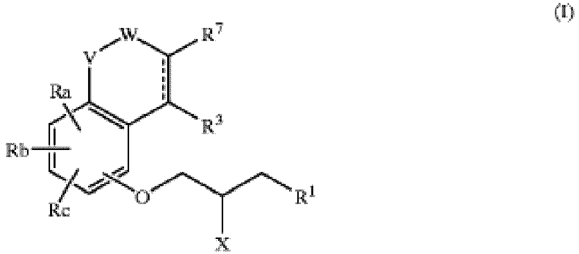
wherein each symbol in the formula means as follows: a bond represented by a solid line and a dotted line shows a double bond or a single bond;
X is a hydrogen atom, a hydroxy group, a C₁ -C₈ alkoxy group, an acyloxy group or an oxo group;
R¹ is a group of the following formula 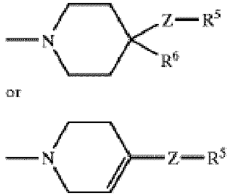
wherein R⁵ is optionally substituted aryl group or optionally substituted aromatic heterocyclic group,
Z is void or --CH₂ --, and
R⁶ is hydrogen atom, hydroxy group, acetamido group, carboxyl group, alkoxycarbonyl group, cyano group or C₁ -C₈ alkoxy group;
R³ is a hydrogen atom, a C₁ -C₁₈ alkyl group or a halogen atom;
V is --O--;
W is void;
R⁷ is a C₁ -C₄ hydroxyalkyl group, an acyl group, an optionally substituted saturated or unsaturated heterocyclic group, an optionally substituted fused heterocyclic group, a C₁ -C₄ alkylsulfonyl group or the formula --Q--R⁹
   wherein
Q is --C(=O)--, --C(=S)--, --CH₂ -- or --S(=O)₂--, and
R⁹ is a group of the following formula 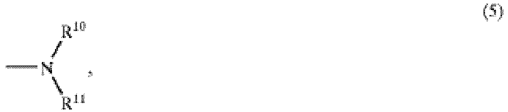 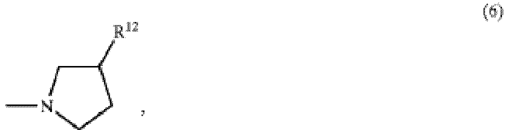 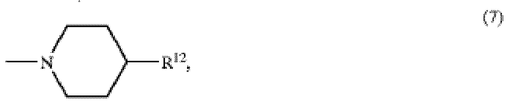 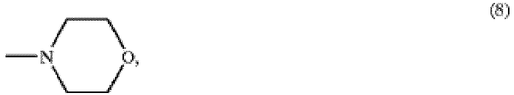 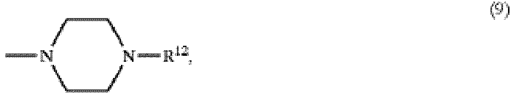 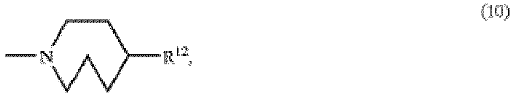 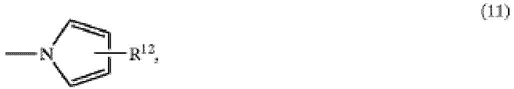 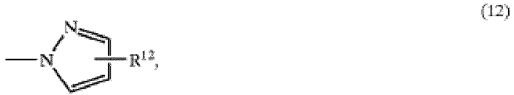 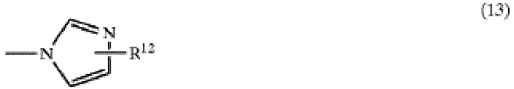
or --NH--NH--R¹⁵
wherein R¹⁰ and R¹¹ are each independently hydrogen atom, C₁ -C₁₈ alkyl group, optionally substituted aryl group, optionally substituted aralkyl group or alkoxy group, R¹² is hydrogen atom, optionally substituted aryl group, C₁ -C₁₈ alkyl group, C₁ -C₈ alkoxy group or acyl group, and R¹⁵ is hydrogen atom, phenyl group, C₁ -C₄ alkyl group, C₁ -C₂ halogenated alkyl group, halogen atom, C₂ -C₄ alkenyl group, C₁ -C₄ hydroxyalkyl group, alkoxyalkyl group, alkyloxycarbonyl group, optionally substituted amino group, acetamido group, carboxyl group, acyl group, optionally substituted alkyloxy group, alkylthio group or cyano group;
Ra, Rb and Rc are each independently a hydrogen atom, a C₁ -C₁₈ alkyl group, a hydroxy group, a C₁ -C₈ alkoxy group, a halogen atom, an acyl group, a nitro group or an amino group;
an optically active compound thereof, a pharmaceutically acceptable salt thereof or a hydrate thereof;
wherein the compound of formula (I) is present in the composition between 0.01 mg/kg and 0.2 mg/kg of the subject's weight and the subject suffers from depression.

In an embodiment, the compound of formula (I) is present in the composition between 0.1 mg and 10 mg. In some embodiments, at least one symptom of depression is also improved or treated in the subject. In some embodiments, the subject has discontinued treatment with an anti-depressant compound.

In other embodiments, as set forth in greater detail elsewhere herein, the dosage and dosing regimen for the compound of formula I may be optimized based on the health and condition of the subject to be treated, as well as the desired outcome of the treatment.

The term "receptor", as used herein, means a molecule, with which one or more kinds of signaling molecules can specifically interact. For example, the 5-HT_{1A} receptor is a subtype of the 5-HT receptor, which binds the neurotransmitter serotonin ("5-hydroxytryptamine").

The term "subject" refers to any animal, including mammals, such as, but not limited to, humans, mice, rats, other rodents, rabbits, dogs, cats, pigs, cattle, sheep, horses, or primates.

The term "treating" (and corresponding terms "treat" and "treatment") includes palliative, restorative, and preventative ("prophylactic") treating of a subject. The term "palliative treating" refers to treatment that eases or reduces the effect or intensity of a condition in a subject without curing the condition. The term "preventative treating" (and the corresponding term "prophylactic treating") refers to treatment that prevents the occurrence of a condition in a subject. The term "restorative treating" ("curative") refers to treatment that halts the progression of, reduces the pathologic manifestations of, or entirely eliminates a condition in a subject. Treating can be done with a therapeutically effective amount of compound, salt or composition that elicits the biological or medicinal response of a tissue, system or subject that is being sought by an individual such as a researcher, doctor, veterinarian, or clinician. The term "treatment" will also be understood to include not only a complete remission of all symptoms experienced by the treated individual, but also the alleviation of one or more existing depressive symptoms, as well as the prevention of occurrence of depressive symptoms by preemptive administration of a compound of formula I to an individual prone to or likely to develop depressive symptoms, such as those with chronic or recurrent depression. The methods of the present disclosure can be used for treatment of any mammal exhibiting symptoms of a depression-related mood disorder, e.g., for treatment of mammals, such as cats, dogs, rats, rabbits, horses and the like; however, in a preferred embodiment, the method is used to treat humans. Preferably, the individual to be treated is one that has been diagnosed with a condition associated with a Major Depressive Episode, more preferably Major Depressive Disorder.

In one aspect of the disclosure encompassed herein, compounds of formula I have been shown to have properties useful to treat depression and/or one or more symptoms of depression.

In another aspect, compounds of formula I are useful for augmenting treatment of depression in a subj ect presently receiving one or more compounds for the treatment of depression.

In an embodiment, a compound of formula I includes the compound set forth in formula II:

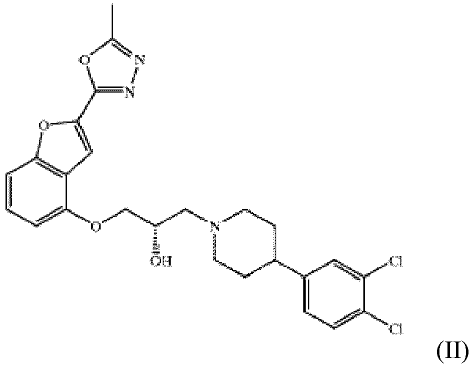

(S)-1-(4-(3,4-dichlorophenyl)piperidino)-3-(2-(5-methyl-1,3,4-oxadiazol-2-yl)benzo(b )furan-4-yloxy)-2-propanol hydrochloride.

In an aspect, a compound of formula II (also referred to herein as SON-117 or Wf-516) has a high affinity for 5-HTT, 5-HT_{1A}, and 5-HT_{2A} receptor binding. Electrophysiological studies using SON-117 demonstrated that the order of effects of SON-117 was presynaptic (i.e., 5-HT_{1A} > 5-HTT > 5-HT_{2A} > postsynaptic 5-HT_{1A}.

In an aspect of the invention, a compound of formula I may have a receptor binding profile with a Kᵢ value of less than 0.05 nmol/L, less than 0.1 nmol/L, less than 0.5 nmol/L, less than 1.0 nmol/L, less than 1.5 nmol/L, less than 2.0 nmol/L, less than 2.5 nmol/L, or less than 5 nmol/L for 5-HTi. In an aspect of the invention, a compound of formula I may have a receptor binding profile with a Kᵢ value of less than 0.05 nmol/L, less than 0.1 nmol/L, less than 0.5 nmol/L, less than 1.0 nmol/L, less than 1.5 nmol/L, less than 2.0 nmol/L, less than 2.5 nmol/L, or less than 5 nmol/L for the dopamine transporter (DAT). As will be understood by the skilled artisan, there may be variation in binding affinities for a compound of formula I when assayed against the same receptor from a different organism or species.

### Associated Symptoms of Depression

Also disclosed herein is a method for treating at least one symptom of a condition or disorder associated with depression in a subject comprising administering to a subject in need thereof a therapeutically effective amount of a compound of the formula (I) or a pharmaceutically acceptable salt, as set forth above. The compound may be the compound set forth in formula II.

### Treatment of Disorders of Sleep

In an embodiment, a method is provided for treating at least one aspect of a disorder of sleep in a subject afflicted with depression, the method comprising administering to a subject in need thereof a therapeutically effective amount of a compound of the formula (I) or a pharmaceutically acceptable salt, as set forth above, wherein the at least one aspect of sleep is treated. In an embodiment, at least one aspect of a disorder of sleep is treated. In another embodiment, at least one aspect or parameter of sleep is improved in a patient. In an embodiment, sleep is improved in a depressed patient. In an embodiment, at least one symptom of depression is improved or treated in conjunction with improvement of sleep in the subject treated in such a manner.

In an aspect, the disruption of at least one disorder or parameter of sleep is associated with depression. However, it will be understood that the present disclosure provides for treatment of at least one disorder or parameter of sleep regardless of how the disorder or affected parameter of sleep arises. In other words, disorders of sleep which are not associated with depression may also be treated according to the disclosure encompassed herein.

In an embodiment of the disclosure, a composition is provided for use in treating or improving at least one disorder or parameter of sleep in a human subject comprising administering to a subject in need thereof a therapeutically effective amount of a composition comprising the compound of formula (I) or a pharmaceutically acceptable salt, hydrate, or solvate thereof, 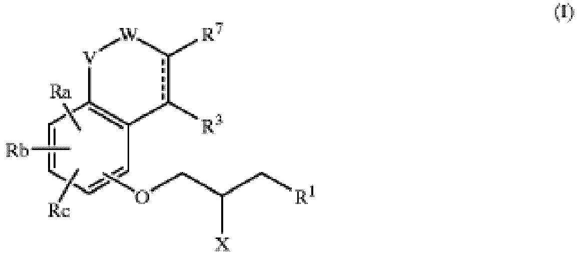
wherein each symbol in the formula means as follows: a bond represented by a solid line and a dotted line shows a double bond or a single bond;
X is a hydrogen atom, a hydroxy group, a C₁ -C₈ alkoxy group, an acyloxy group or an oxo group;
R¹ is a group of the following formula

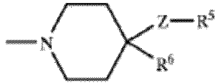

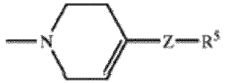

wherein R⁵ is optionally substituted aryl group or optionally substituted aromatic heterocyclic group,
Z is void or --CH₂ --, and
R⁶ is hydrogen atom, hydroxy group, acetamido group, carboxyl group, alkoxycarbonyl group, cyano group or C₁ -C₈ alkoxy group;
R³ is a hydrogen atom, a C₁ -C₁₈ alkyl group or a halogen atom;
V is --O--;
W is void;
R⁷ is a C₁ -C₄ hydroxyalkyl group, an acyl group, an optionally substituted saturated or unsaturated heterocyclic group, an optionally substituted fused heterocyclic group, a C₁ -C₄ alkylsulfonyl group or the formula --Q--R⁹
   wherein
Q is --C(=O)--, --C(=S)--, --CH₂ -- or --S(=O)₂--, and
R⁹ is a group of the following formula

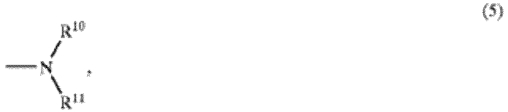

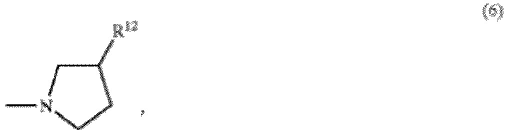

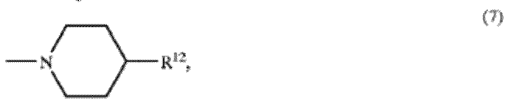

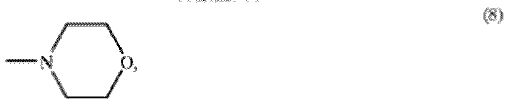

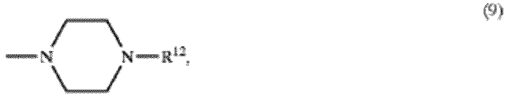

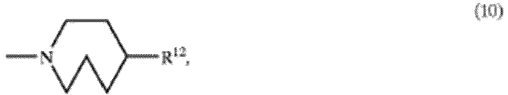

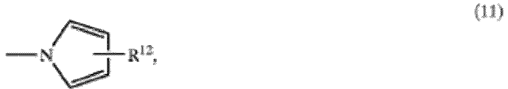

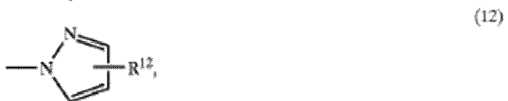

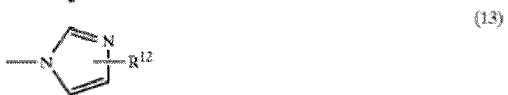

or --NH--NH--R¹⁵
wherein R¹⁰ and R¹¹ are each independently hydrogen atom, C₁ -C₁₈ alkyl group, optionally substituted aryl group, optionally substituted aralkyl group or alkoxy group, R¹² is hydrogen atom, optionally substituted aryl group, C₁ -C₁₈ alkyl group, C₁ -C₈ alkoxy group or acyl group, and R¹⁵ is hydrogen atom, phenyl group, C₁-C₄ alkyl group, C₁-C₂ halogenated alkyl group, halogen atom, C₂-C₄ alkenyl group, C₁-C₄ hydroxyalkyl group, alkoxyalkyl group, alkyloxycarbonyl group, optionally substituted amino group, acetamido group, carboxyl group, acyl group, optionally substituted alkyloxy group, alkylthio group or cyano group;
Ra, Rb and Rc are each independently a hydrogen atom, a C₁ -C₁₈ alkyl group, a hydroxy group, a C₁ -C₈ alkoxy group, a halogen atom, an acyl group, a nitro group or an amino group;
an optically active compound thereof, a pharmaceutically acceptable salt thereof or a hydrate thereof;
wherein the compound of formula (I) is present in the composition between 0.01 mg/kg and 0.2 mg/kg of the subject's weight.

In an embodiment, the compound of formula (I) is present in the composition between 0.1 mg and 10 mg.

In an embodiment, the compound of formula I is the compound set forth in formula II.

In accordance with the present invention, the subject being treated for a disorder of sleep with a compound of formula I also suffers from depression.

In an embodiment of the disclosure, sleep is improved in a patient who is not afflicted with depression. In an aspect, at least one disorder or parameter of sleep is treated and/or improved. In an aspect, a method is provided for improving at least one aspect of sleep, comprising administering to a subject in need thereof a therapeutically effective amount of a compound of the formula (I) or a pharmaceutically acceptable salt, as set forth above. In an embodiment, the compound of formula I is the compound set forth in formula II.

In one embodiment, a method is provided for treating a sleep disorder in a patient afflicted with depression. In one embodiment, a method is provided for treating a sleep disorder in a patient following discontinuation of treatment with another active pharmaceutical ingredient, for example, an anti-depressant compound. In one embodiment, a method is provided for treating a sleep disorder in combination with an active pharmaceutical ingredient (for example, an anti-depressant compound).

Various aspects of sleep may be treated, including, but not limited to, sleep onset latency, latency to persistent sleep, distribution of slow wave sleep across the sleep period time, or one or more segments of sleep period time, overall sleep continuity and sleep architecture parameters. In an aspect, slow wave sleep (SWS) is increased, even at the low dose of 1 mg, when the compound is SON-117. In an aspect, REM activity and REM density are increased, even at the low doses of 3 mg and 7.5 mg, when the compound is SON-117. These effects are distinct and unexpected compared to the effect of other mono-aminergic antidepressants, such as selective serotonin reuptake inhibitors (SSRIs) or serotonin-norepinephrine reuptake inhibitors (SNRIs).

Cognitive impairment is the diminished ability to think, concentrate, formulate ideas, reason and remember. In an embodiment of the disclosure, a method is provided for treating or diminishing cognitive impairment or improving cognition, comprising administering to a subject in need thereof a therapeutically effective amount of a compound of the formula (I) or a pharmaceutically acceptable salt, as set forth herein. In one embodiment of the disclosure, a method is provided for treating depression without provoking cognitive impairment. In one embodiment of the disclosure, a method is provided for treating depression and restoring, enhancing, and improving cognition, in a patient following discontinuation of treatment with another active pharmaceutical ingredient, for example, an anti-depressant compound. In one embodiment of the disclosure, a method is provided for treating depression in combination with a cognition impairing active pharmaceutical ingredient (for example, a cognition impairing anti-depressant compound), without causing or increasing cognitive impairment, or for improving, enhancing or restoring cognition in such a patient. In an embodiment of the disclosure, there is no loss of cognition in a person suffering from depression by the administration of SON-117 as set forth herein. In another embodiment of the disclosure, cognitive impairment present in a person suffering from depression is treated or diminished by the administration of SON-117 as set forth herein. In an embodiment of the disclosure, the compound of formula I is the compound set forth in formula II. As will be understood based on the disclosure herein, modification of sleep parameters can improve cognition. By way of a non-limiting example, improvement and/or an increase in SWS improves cognition. In an aspect, cognition in general is improved. In another aspect, one or more aspects of cognition are improved, including, among others, memory consolidation, executive functions, verbal memory, and verbal fluency. In an embodiment of the disclosure, cognition is improved in a subject to the point where normal cognition is restored in the subject. In another embodiment of the disclosure, cognition is improved in a subject beyond the point of normal cognition in the subject, such that levels of cognition in the subject are enhanced.

In an embodiment of the disclosure, embodiment, cognition is improved in a subject afflicted with depression. In another embodiment of the disclosure, cognition is improved in a subject not afflicted with depression

### Treatment of Other Disorders

In an embodiment of the disclosure, a method is provided for treating the elderly using a composition encompassed herein. In one embodiment of the disclosure, an elderly subject has a pre-Alzheimer's Disease condition or state. In one embodiment of the disclosure, an elderly subject has a pre-Parkinson's Disease condition or state.

In an embodiment of the disclosure, a method of treating the elderly using a composition encompassed herein comprises treatment of depression, and may optionally comprise co-administration with one or more additional therapeutic agents, as described in greater detail elsewhere herein. In an embodiment of the disclosure, a method of treating the elderly using a composition encompassed herein comprises treatment of depression, and may optionally comprise co-administration with one or more additional therapeutic agents, such as, but not limited to, citalopram, escitalopram, sertraline, buproprion, mirtazapine, moclobemide, venlafaxine, desipramine, and nortriptyline.

In an embodiment of the disclosure, patients who suffer from increased sexual dysfunction (such as, for example, anorgasmia and delayed ejaculation) due to the effects of the antidepressive drug that they are receiving or the depression itself, experience decreased dysfunction or even an improvement in the dysfunction may occur when subsequently dosed with SON-117 as set forth herein.

In one embodiment of the disclosure, a method is provided for treating depression without causing or increasing sexual dysfunction. In one embodiment of the disclosure, a method is provided for treating depression in combination with an active pharmaceutical ingredient (for example, an anti-depressant compound), without causing or increasing sexual dysfunction in a patient.

### Dosage Forms and Amounts

In an embodiment, as set forth in detail elsewhere herein, it has now been found that low doses of a compound of formula II administered to a subject provide surprising efficacy for treatment of depression. In an aspect, a disorder of sleep is unexpectedly treated in a subject to which a low dose of the compound of formula II is administered. In another aspect, cognition is unexpectedly improved in a subject to which a low dose of the compound of formula II is administered. In an embodiment, a compound of formula I is administered to a subject at a dose of less than 0.01 mg/kg. In an embodiment, the compound is administered parenterally at a dose of less than 0.01 mg/kg. In an embodiment, the compound is a compound of formula II. In an embodiment, a compound of formula I is administered to a subject at a dose of less than 0.5 mg/kg. In an embodiment, the compound is administered orally at a dose of less than 0.5 mg/kg. In an embodiment, the compound is a compound of formula II.

In an embodiment, it has also been found that the onset of activity of a compound of formula I (e.g., the compound of formula II) is rapid, and occurs much more rapidly than for conventional antidepressant compounds known in the art.

In an embodiment, a dose encompassed herein may be administered as a composition based on the weight of the subject. In an embodiment, a dose may be administered per unit weight of the subject (e.g., mg compound of formula I per kg weight of subject). In an embodiment, a dose encompassed herein may be administered as a composition based solely on the weight of the dose, without regard to the weight of the subject (e.g., mg of compound of formula I per dose administered to subject). In an embodiment, the dose is determined based on the weight of the compound of formula I in the dosage form. In another embodiment, the dose is determined based on the total weight of all components of the composition comprising the dosage form.

In an embodiment, administration of a compound for any purpose as described herein, in any form or combination described herein, may include administering the compound of formula I or a pharmaceutically acceptable salt thereof at a dose of between 1 ng - 100 µg, 5 ng - 75 µg, 10 ng - 50 µg, 25 ng - 40 µg, 50 ng - 30 µg, 75 ng - 20 µg, 100 ng - 10 µg, 250 ng - 5 µg, 500 ng - 200 µg, 750 ng - 100 µg, 1 µg - 75 µg, 5 µg - 50 µg, or 10 µg - 40 µg. By way of a non-limiting example, and as set forth in detail elsewhere herein, a dose of 10 µg - 40 µg, for example, represents a dosage range of 10 µg per kg of subject weight to 40 µg per kg of subject weight, and can also represent a dosage range of 10 µg administered to a subject to 40 µg administered to a subj ect.

In an embodiment, administration of a compound for any purpose as described herein, in any form or combination described herein, may include administering the compound of formula I or a pharmaceutically acceptable salt thereof at a dose of between 1 ng - 1 g, 5 ng - 1 g, 10 ng - 1 g, 25 ng - 1 g, 50 ng - 1 g, 75 ng - 1 g, 100 ng - 750 mg, 500 ng - 500 mg, 10 µg - 200 mg, 15 µg - 190 mg, 25 µg - 180 mg, 50 µg - 170 mg, 75 µg - 160 mg, 100 µg - 150 mg, 250 µg - 140 mg, 400 µg - 130 mg, between 500 µg - 128 mg, 600 µg - 100 mg, 750 µg - 75 mg, 900 µg - 50 mg, or at a dose between 0.1 mg - 64 mg.

In an embodiment, administration of a compound for any purpose as described herein, in any form or combination described herein, may include administering the compound of formula I or a pharmaceutically acceptable salt thereof at a dose of between 0.01 - 1.0 mg, 0.05 - 0.75 mg, 0.08 - 0.6 mg, 0.01 - 0.2 mg, 0.05 mg - 0.15 mg, 0.08 - 0.12 mg, 0.4 - 0.6 mg, 0.45 - 0.55 mg, or at a dose between 0.48 - 0.52 mg.

In an embodiment, a compound of formula I or a pharmaceutically acceptable salt thereof may be administered to a subject according to the compositions and methods encompassed herein at a dose of about 1000 mg or less, about 500 mg or less, about 200 mg or less, about 150 mg or less, about 100 mg or less, about 50 mg or less, about 40 mg or less, about 30 mg or less, about 20 mg or less, about 10 mg or less, about 9 mg or less, about 8 mg or less, about 7 mg or less, about 6 mg or less, about 5 mg or less, about 4 mg or less, about 3 mg or less, about 2 mg or less, about 1 mg or less, about 0.9 mg or less, about 0.8 mg or less, about 0.7 mg or less, about 0.6 mg or less, about 0.5 mg or less, about 0.4 mg or less, about 0.3 mg or less, about 0.2 mg or less, about 0.1 mg or less, about 0.09 mg or less, about 0.08 mg or less, about 0.07 mg or less, about 0.06 mg or less, about 0.05 mg or less, about 0.04 mg or less, about 0.03 mg or less, about 0.02 mg or less, about 0.01 mg or less, about 0.009 mg or less, about 0.008 mg or less, about 0.007 mg or less, about 0.006 mg or less, about 0.005 mg or less, about 0.004 mg or less, about 0.003 mg or less, about 0.002 mg or less, about 0.001 mg or less, or about 0.0005 mg or less.

In an embodiment, a compound of formula I or a pharmaceutically acceptable salt thereof may be administered to a subject according to the compositions and methods encompassed herein at a dose of about 1 g, about 500 mg or more, about 200 mg or more, about 150 mg or more, about 100 mg or more, about 50 mg or more, about 40 mg or more, about 30 mg or more, about 20 mg or more, about 10 mg or more, about 9 mg or more, about 8 mg or more, about 7 mg or more, about 6 mg or more, about 5 mg or more, about 4 mg or more, about 3 mg or more, about 2 mg or more, about 1 mg or more, about 0.9 mg or more, about 0.8 mg or more, about 0.7 mg or more, about 0.6 mg or more, about 0.5 mg or more, about 0.4 mg or more, about 0.3 mg or more, about 0.2 mg or more, about 0.1 mg or more, about 0.09 mg or more, about 0.08 mg or more, about 0.07 mg or more, about 0.06 mg or more, about 0.05 mg or more, about 0.04 mg or more, about 0.03 mg or more, about 0.02 mg or more, about 0.01 mg or more, about 0.009 mg or more, about 0.008 mg or more, about 0.007 mg or more, about 0.006 mg or more, about 0.005 mg or more, about 0.004 mg or more, about 0.003 mg or more, about 0.002 mg or more, about 0.001 mg or more, or about 0.0005 mg or more.

In an embodiment, a compound of formula I or a pharmaceutically acceptable salt thereof may be administered to a subject according to the compositions and methods encompassed herein at a dose that combines any of the foregoing dose ranges. As a non-limiting example, in an embodiment, a compound of formula I or a pharmaceutically acceptable salt thereof may be administered to a subject according to the compositions and methods encompassed herein at a dose of about 0.1 mg or more and about 0.5 mg or less.

In an embodiment, a compound of formula I or a pharmaceutically acceptable salt thereof may be administered to a subject according to the compositions and methods encompassed herein at a dose of about 1000 mg, about 500 mg, about 200 mg, about 150 mg, about 100 mg, about 50 mg, about 40 mg, about 30 mg, about 20 mg, about 10 mg, about 9 mg, about 8 mg, about 7 mg, about 6 mg, about 5 mg, about 4 mg, about 3 mg, about 2 mg, about 1 mg, about 0.9 mg, about 0.8 mg, about 0.7 mg, about 0.6 mg, about 0.5 mg, about 0.4 mg, about 0.3 mg, about 0.2 mg, about 0.1 mg, about 0.09 mg, about 0.08 mg, about 0.07 mg, about 0.06 mg, about 0.05 mg, about 0.04 mg, about 0.03 mg, about 0.02 mg, about 0.01 mg, about 0.009 mg, about 0.008 mg, about 0.007 mg, about 0.006 mg, about 0.005 mg, about 0.004 mg, about 0.003 mg, about 0.002 mg, about 0.001 mg, or about 0.0005 mg.

In an embodiment, a compound of formula I or a pharmaceutically acceptable salt thereof may be administered to a subject according to the compositions and methods encompassed herein at a dose of 1000 mg, 500 mg, 200 mg, 150 mg, 100 mg, 50 mg, 40 mg, 30 mg, 20 mg, 10 mg, 9 mg, 8 mg, 7 mg, 6 mg, 5 mg, 4 mg, 3 mg, 2 mg, 1.9 mg, 1.8 mg, 1.7 mg, 1.6 mg, 1.5 mg, 1.4 mg, 1.3 mg, 1.2 mg, 1.1 mg, 1 mg, 0.9 mg, 0.8 mg, 0.7 mg, 0.6 mg, 0.5 mg, 0.4 mg, 0.3 mg, 0.2 mg, 0.1 mg, 0.09 mg, 0.08 mg, 0.07 mg, 0.06 mg, 0.05 mg, 0.04 mg, 0.03 mg, 0.02 mg, 0.01 mg, 0.009 mg, 0.008 mg, 0.007 mg, 0.006 mg, 0.005 mg, 0.004 mg, 0.003 mg, 0.002 mg, 0.001 mg, or 0.0005 mg.

In an embodiment, a compound of formula I or a pharmaceutically acceptable salt thereof may be administered to a subject according to the compositions and methods encompassed herein at a dose of about 1000 mg/kg, about 500 mg/kg, about 200 mg/kg, about 150 mg/kg, about 100 mg/kg, about 50 mg/kg, about 40 mg/kg, about 30 mg/kg, about 20 mg/kg, about 10 mg/kg, about 9 mg/kg, about 8 mg/kg, about 7 mg/kg, about 6 mg/kg, about 5 mg/kg, about 4 mg/kg, about 3 mg/kg, about 2 mg/kg, about 1.9 mg/kg, about 1.8 mg/kg, about 1.7 mg/kg, about 1.6 mg/kg, about 1.5 mg/kg, about 1.4 mg/kg, about 1.3 mg/kg, about 1.2 mg/kg, about 1.1 mg/kg, about 1 mg/kg, about 0.9 mg/kg, about 0.8 mg/kg, about 0.7 mg/kg, about 0.6 mg/kg, about 0.5 mg/kg, about 0.4 mg/kg, about 0.3 mg/kg, about 0.2 mg/kg, about 0.1 mg/kg, about 0.09 mg/kg, about 0.08 mg/kg, about 0.07 mg/kg, about 0.06 mg, about 0.05 mg, about 0.04 mg, about 0.03 mg/kg, about 0.02 mg, about 0.01 mg, about 0.009 mg, about 0.008 mg, about 0.007 mg, about 0.006 mg/kg, about 0.005 mg/kg, about 0.004 mg/kg, about 0.003 mg/kg, about 0.002 mg/kg, about 0.0019 mg/kg, about 0.0018 mg/kg, about 0.0017 mg/kg, about 0.0016 mg/kg, about 0.0015 mg/kg, about 0.0014 mg/kg, about 0.0013 mg/kg, about 0.0012 mg/kg, about 0.0011 mg/kg, about 0.001 mg/kg, about 0.0005 mg/kg, or any range determinable from the preceding dosages (e.g., about .0013 mg/kg to about .0016 mg/kg or about .001 mg/kg to about .002 mg/kg).

In an aspect, a method of administering a compound of formula I may include titration of the compound up to a predetermined level. In one embodiment, a compound is used at a specified level (E.g., 0.05 mg b.i.d., 0.1 mg b.i.d., 0.2 mg b.i.d., 0.4 mg b.i.d., 0.6 mg b.i.d., 0.8 mg b.i.d., 1 mg b.i.d., 2 mg b.i.d., 4 mg b.i.d., 8 mg b.i.d., 16 mg b.i.d., 32 mg b.i.d., 64 mg b.i.d.). In one embodiment, a compound is used at a specified level (E.g., about 0.05 mg b.i.d., about 0.1 mg b.i.d., about 0.2 mg b.i.d., about 0.4 mg b.i.d., about 0.6 mg b.i.d., about 0.8 mg b.i.d., about 1 mg b.i.d., about 2 mg b.i.d., about 4 mg b.i.d., about 8 mg b.i.d., about 16 mg b.i.d., 32 mg b.i.d., 64 mg b.i.d.). In one embodiment, the compound is titrated up to a predetermined dosage (E.g., titration up to 0.1 mg b.i.d., 0.2 mg b.i.d., 0.4 mg b.i.d., 0.6 mg b.i.d., 0.8 mg b.i.d., 1 mg b.i.d., 2 mg b.i.d., 4 mg b.i.d., 8 mg b.i.d., 16 mg b.i.d., 32 mg b.i.d., 64 mg b.i.d., etc...). In one embodiment, the compound is titrated up to a predetermined dosage (E.g., titration up to about 0.1 mg b.i.d., about 0.2 mg b.i.d., about 0.4 mg b.i.d., about 0.6 mg b.i.d., about 0.8 mg b.i.d., about 1 mg b.i.d., about 2 mg b.i.d., about 4 mg b.i.d., about 8 mg b.i.d., about 16 mg b.i.d., about 32 mg b.i.d., about 64 mg b.i.d., etc...). In an embodiment, a compound is titrated up to a predetermined dosage as the dosage is described elsewhere herein.

In an embodiment, a compound of formula I may be used and/or administered to a subject based a desired plasma concentration of the compound. In an embodiment, the dosage of compound administered to a subject is determined by identifying the dosage required to obtain a plasma concentration of about 10 ng/ml of the compound of formula I in a subject. In an embodiment, the dosage of compound administered to a subject is determined by identifying the dosage required to obtain a plasma concentration of about 1 ng/ml, about 2 ng/ml, about 3 ng/ml, about 4 ng/ml, about 5 ng/ml, about 6 ng/ml, about 7 ng/ml, about 8 ng/ml, about 9 ng/ml, about 10 ng/ml, about 12 ng/ml, about 14 ng/ml, about 16 ng/ml, about 18 ng/ml, about 20 ng/ml, about 25 ng/ml, about 30 ng/ml, about 35 ng/ml, about 40 ng/ml, about 45 ng/ml, or about 50 ng/ml of the compound of formula I in a subject.

The dose may be administered as a weekly dose, a single daily dose, twice daily, three times daily, four times daily, five times daily, or more frequently. In an embodiment, administration frequency may be between 1 and 5 times a day. In another embodiment, administration frequency may be between 2 and 4 times a day. In another embodiment, administration frequency may be at least 3 times a day. In another embodiment, administration frequency may be twice a day. In another embodiment, administration frequency may be once a day. In another embodiment, administration frequency may be less frequent than once a day. In other embodiments, administration frequency may be once every 2 days or once every 3 days or once every 4 days or once every 5 days or once every 6 days. In another embodiment, administration frequency may be once a week. In another embodiment, administration frequency may be on demand, as therapeutic treatment is required or desired. It will be understood, based on the disclosure encompassed herein, how to determine whether a subject needs an additional and/or continued dose. It will also be understood that the selected dosing frequency may require an adjustment of the dosage of active ingredient. It will also be understood, based on the disclosure encompassed herein, that the selected dosage of active ingredient may require an adjustment of the dosing frequency. The disclosure encompassed herein, in combination with the skill in the art, will enable the skilled artisan to optimize both the dosage of a compound of formula I and the frequency of administration of the compound of formula I to a treat depression in a subject in need thereof.

It will further be understood by the skilled artisan that, in addition to the above embodiments of dosage and dosing regimens, both the dosage and the dosing regimen will be considered and each adjusted, as necessary, in view of the physical and mental health of the subject being treated for depression, including the severity of the subject's depression.

For therapeutic administration according to the present invention, a compound of formula I may be employed in the form of its free base, but is preferably used in the form of a pharmaceutically acceptable salt, typically the hydrochloride salt.

In an embodiment, alternative salts of a compound of formula I with pharmaceutically acceptable acids may also be utilized in therapeutic administration, for example salts derived from the functional free base and acids including, but not limited to, palmitic acid, hydrobromic acid, phosphoric acid, acetic acid, fumaric acid, maleic acid, salicylic acid, citric acid, oxalic acid, lactic acid, malic acid, methanesulphonic acid and p-toluene sulphonic acid.

All solvates and all alternative physical forms of a compound of formula I or its pharmaceutically acceptable derivatives as described herein, including but not limited to alternative crystalline forms, amorphous forms and polymorphs, are also within the scope of this invention, and all references to a compound of formula I herein include all pharmaceutically acceptable salts, and all solvates and alternative physical forms thereof.

For therapeutic administration, a compound of formula I or a pharmaceutically acceptable salt thereof, for example, the compound of formula II, may be administered in pure form, but will preferably be formulated into any suitable pharmaceutically acceptable and effective composition which provides effective levels of the active ingredient in the body.

The term "pharmaceutically acceptable", as used herein with respect to a compound or composition, refers to a form of the compound or composition that can increase or enhance the solubility or availability of the compound in a subject, in order to promote or enhance the bioavailability of the compound or composition. In an aspect, the disclosure herein also encompasses pharmaceutically acceptable, hydrates, solvates, stereoisomers, or amorphous solids of the compounds and compositions embodied herein. For example, the term "pharmaceutically acceptable salt" is to describe a salt form of one or more of the compositions herein which are presented to increase the solubility of the compound, for example, in the gastric juices of the patient's gastrointestinal tract in order to promote dissolution and the bioavailability of the compounds and/or compositions. In an embodiment, pharmaceutically acceptable salts include those derived from pharmaceutically acceptable inorganic or organic bases and acids. Suitable salts include those derived from alkali metals such as potassium and sodium, alkaline earth metals such as calcium, magnesium and ammonium salts, among numerous other acids well known in the pharmaceutical art. Sodium and potassium salts are particularly preferred as neutralization salts of carboxylic acids and free acid phosphate containing compositions encompassed by the present disclosure. The term "salt" shall mean any salt consistent with the use of the compounds encompassed by the present disclosure. In the case where the compounds are used in pharmaceutical indications, including the treatment of depression, the term "salt" shall mean a pharmaceutically acceptable salt, consistent with the use of the compounds as pharmaceutical agents.

The term "pharmaceutically acceptable derivative" or "derivative", as used herein, describes any pharmaceutically acceptable prodrug form (such as an ester or ether or other prodrug group) which, upon administration to a patient, provides directly or indirectly the present compound or an active metabolite of the present compound.

As set forth above, the compositions include pharmaceutically acceptable salts of the compounds in the composition. In other embodiments, the acids which are used to prepare the pharmaceutically acceptable acid addition salts of the aforementioned compounds are those which form non-toxic acid addition salts, i.e., salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [i.e., 1,1'-methylene-bis-(2-hydroxy-3 naphthoate)] salts, among others.

In an embodiment, compositions comprise base addition salts of the present compounds. The chemical bases that may be used as reagents to prepare pharmaceutically acceptable base salts of the present compounds that are acidic in nature are those that form non-toxic base salts with such compounds. Such non-toxic base salts include, but are not limited to those derived from such pharmacologically acceptable cations such as alkali metal cations (e.g., potassium and sodium) and alkaline earth metal cations (e.g., calcium and magnesium), ammonium or water-soluble amine addition salts such as N-methylglucamine (meglumine), and the lower alkanolammonium and other base salts of pharmaceutically acceptable organic amines, among others.

As used herein, the term pharmaceutically acceptable salts or complexes refers to salts or complexes (e.g., solvates, polymorphs) that retain the desired biological activity of the parent compound and exhibit minimal, if any, undesired toxicological effects. Nonlimiting examples of such salts are (a) acid addition salts formed with inorganic acids (for example, hydrochloric acid, hydrobromic acid, sulfuric acid, phosphoric acid, nitric acid, and the like), and salts formed with organic acids such as acetic acid, oxalic acid, tartaric acid, succinic acid, malic acid, ascorbic acid, benzoic acid, tannic acid, pamoic acid, alginic acid, polyglutamic acid, naphthalenesulfonic acids, naphthalenedisulfonic acids, and polygalacturonic acid; (b) base addition salts formed with polyvalent metal cations such as zinc, calcium, bismuth, barium, magnesium, aluminum, copper, cobalt, nickel, cadmium, sodium, potassium, and the like, or with an organic cation formed from N,N-dibenzylethylene-diamine, ammonium, or ethylenediamine; or (c) combinations of (a) and (b); e.g., a zinc tannate salt or the like.

Modifications of a compound can affect the solubility, bioavailability and rate of metabolism of the active species, thus providing control over the delivery of the active species. Further, the modifications can affect the antidepressant activity of the compound, in some cases increasing the activity over the parent compound. This can easily be assessed by preparing the derivative and testing its antidepressant activity according to the methods encompassed herein, or other methods known to those skilled in the art.

In an embodiment, the compositions may be formulated in a conventional manner using one or more pharmaceutically acceptable carriers and may also be administered in controlled-release formulations. Pharmaceutically acceptable carriers that may be used in these pharmaceutical compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as prolamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

Compositions encompassed herein may be administered orally. In other embodiments, compositions may be administered parenterally, by inhalation spray, topically, rectally, nasally, buccally, vaginally or via an implanted reservoir. The term "parenteral" as used herein includes subcutaneous, percutaneous, intravenous, intramuscular, intra-articular, intra-synovial, intrasternal, intrathecal, intrahepatic, intralesional and intracranial injection or infusion techniques. As will be understood by the skilled artisan, in view of the embodiments encompassed herein, the dosage of active ingredient or ingredients (e.g., a compound of formula I) may be adjusted upward or downward based on the selected route of administration. Furthermore, it will be understood that optimizing the dosage of active ingredient for any selected dosage form may be desired and can be achieved by using the methods described herein or known in the art to evaluate the effectiveness of antidepressant compounds.

The pharmaceutical compositions embodied herein may be orally administered in any orally acceptable dosage form including, but not limited to, capsules, tablets, aqueous suspensions or solutions. In the case of tablets for oral use, carriers which are commonly used include lactose and corn starch. In an embodiment, lubricating agents, such as magnesium stearate, are also added. For oral administration in a capsule form, useful diluents include lactose and/or dried corn starch, as two non-limiting examples. When aqueous suspensions are required for oral use, the active ingredient is combined with emulsifying and suspending agents. If desired, certain sweetening, flavoring or coloring agents may also be added.

The pharmaceutical compositions encompassed by the present disclosure may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

In an embodiment, a compound of formula I or a pharmaceutically acceptable salt thereof is administered independently of any other medication.

### Co-Administration of Compounds

In another embodiment, a compound of formula I or a pharmaceutically acceptable salt thereof is administered in conjunction with one or more other medications. Such other medications may be administered or co-administered in forms and dosages as known in the art, or in the alternative, as has been described above for administration of compounds of formula I.

The term "coadministration" or "combination therapy" is used to describe a therapy in which at least two compounds are used to treat depression or another disease state or condition as described herein at the same time. In an embodiment, at least two compounds in effective amounts are used to treat depression or another disease state or condition as described herein at the same time. In another embodiment, at least two compounds, the combination of which comprises an effective amount, are used to treat depression or another disease state or condition as described herein at the same time. In an embodiment, the result of treatment with the at least two compounds may be additive of the treatment results obtained using each compound separately, either directly additive, or additive to a degree lesser than the results obtained with the two compounds separately. In an embodiment, the result of treatment with the at least two compounds may be synergistic, to varying degrees. In an embodiment, the result of treatment with the at least two compounds may be less than the treatment results obtained using each compound separately. In an aspect, the result of treatment with a composition encompassed herein is such that, for one compound, the result of treatment is less than that obtained with the compound separately, while the results of treatment with respect to the other compounds in the composition are about the same as the results of treatment obtained separately. In an aspect, the result of treatment for at least two compounds is less than that obtained with the compounds separately, while the other compounds in the composition are about the same as the results of treatment obtained separately. In an aspect, the result of treatment for all compounds in the composition is less than that obtained with the compounds separately.

Although the term coadministration encompasses the administration of two active compounds to the patient at the same time, it is not necessary that the compounds be administered to the patient at the same time, although effective amounts of the individual compounds will be present in the patient at the same time.

A compound of formula I, for example, the compound set forth in formula II, or a pharmaceutically acceptable salt of any such compound, may advantageously be administered in combination with at least one other therapeutic agent to provide improved treatment of any combination of any symptoms of depression and/or the treatment of depression itself. The combinations, uses and methods of treatment of the invention may also provide advantages in treatment of patients who fail to respond adequately or who are resistant to other known treatments. In an embodiment, the at least one other therapeutic agent is not a compound of formula I. In an embodiment, the at least one other therapeutic agent is a compound of formula I, and is different than the other compound of formula I included in the composition. In an embodiment, the compound of formula II is coadministered with at least one other compound of formula I, wherein the at least one other compound of formula I is not formula II. In an embodiment, the compound of formula II is coadministered with at least one other compound, wherein the at least one other compound is not a compound of formula I.

In an embodiment, a compound of formula I may be administered to a patient already undergoing treatment with at least one antidepressant, to provide improved treatment of any combination of negative symptoms of depression, or the treatment of depression itself.

In an embodiment, a compound set forth herein can be coadministered with one or more atypical antipsychotics. Examples of atypical antipsychotics include, but are not limited to fluphenazine, risperidone, olanzapine, clozapine, quetiapine, ziprasidone, aripiprazole, seritindole, zotepine, and perospirone. Examples of antidepressants useful in combination therapy as encompassed herein include, but are not limited to, fluoxetine, citalopram, escitalopram, venlafaxine, duloxetine, bupropion.

In an embodiment, a compound set forth herein can be coadministered with one or more anxiolytics. In one embodiment, a compound set forth herein is coadministered with propranolol. In an aspect, a co-administered compound can enhance the onset of activity of a compound encompassed herein. In an embodiment, a beta-blocker is coadministered with a compound encompassed herein to enhance the onset of activity of a compound encompassed herein.

The methods and dosages described herein are applicable to methods of use and dosages of compounds of formula I and formula II herein.

### EXAMPLES

The embodiments encompassed herein are now described with reference to the following Examples. These Examples are provided for the purpose of illustration only and the disclosure encompassed herein should in no way be construed as being limited to these Examples, but rather should be construed to encompass any and all variations which become evident as a result of the teachings provided herein.

### Example 1: Effect of SON-117 on Sleep Parameters in Healthy Volunteers

In this study, certain pharmacodynamic sleep parameters predictive of antidepressant activity were monitored and measured. The positive comparator (escitalopram, Celexa) behaved as expected. Three doses of SON-117 were tested (1 mg, 3 mg, 7.5 mg). Each dose exhibited a different pharmacodynamic profile than escitalopram and placebo. In addition, the lowest dose of SON-117 (1 mg) demonstrated a different profile than the two higher doses of SON-117. In an embodiment, SON-117 affected sleep. In an embodiment, SON-117 affected REM sleep.

While not wishing to be bound by any particular theory, one possible mode of action (among other possible mode of action) may be that higher doses of SON-117 contact and/or affect receptors that low doses of SON-117 do not modulate.

### Example 2: Polysomnography

Forty-four (44) subjects were tested in a sleep study, where 23 subjects were dosed with SON-117 in amounts of 1 mg up to 7.5 mg; 12 were given a placebo; and 8 were given escitalopram at a dose of 20 mg. Sleep was recorded in individual sound-attenuated and comfortably furnished bedrooms. During bedtime hours subjects were recumbent and the lights are turned off. The polysomnographic montage consisted of 4 EEG channels (C3A2, O1A2, C4A1, and O2A1), bilateral electro-oculograms, and 2 submental electromyograms. The different sleep parameters were derived with the Hypnos software from the visual scoring of the recordings at 30 sec epochs according to Rechtschaffen and Kales (1968) rules. Sleep continuity parameters comprise both sleep initiation and sleep maintenance variables. Sleep architecture parameters, which include REM sleep parameters, comprise stages distribution variables documenting duration and proportion of the different sleep stages and sleep profile variables that provide an outline on the time course of the different sleep stages during the recording period. All night sleep EEG spectral analysis was carried out on the C3-A2 derivation by means a fast Fourrier transform algorithm. Epoch length was 2 seconds at a sampling rate of 256 Hz and truncating error was reduced by applying a Hanning window. Sleep visual scores of each 30-second epoch was synchronised with power density values in order to allow the analysis of power density within in specific period of time such as REM and non REM sleep.

The study failed to find an effect on sleep EEG parameters of SON-117 that would be comparable with those induced by escitalopram. Sleep continuity and sleep architecture parameters were largely not influenced by the three doses tested in the present study. However, there were indications that the drug could have REM sleep promoting effect, as well as effects on REM density/activity, with repeated dosing and at the two highest dosages, particularly (7.5mg). Furthermore, the lowest dose (1 mg) could increase slow wave sleep (SWS) at day 1 and at day 14 in the second third of the night. These results on SWS are reinforced by the fact that Leeds Sleep Evaluation Questionnaire (LSEQ) getting to sleep was rated as easier with 1 mg of SON-117 and that LSEQ quality of sleep was found improved. These subjective effects on sleep were observed after a single administration of 1 mg of SON-117 and were sustained up to the last observational time point, i.e. at day 16 after discontinuation of dosing. There was no evidence of discontinuation of dosing on sleep EEG parameters, including on REM sleep.

### Example 3: Measurement of Neurotransmitters in Rats Treated With SON-117

The aim of the study was to evaluate, using microdialysis, the effects of an intraperitoneal (I.P.) administration in rats of three different doses of SON-117 on dopamine and its metabolites, serotonin and its metabolite (5-HIAA), and norepinephrine levels in prefrontal cortex.

Thirty-two adult male Wistar rats were used for the whole study (4 groups of eight animals planned for the study, Table 1).

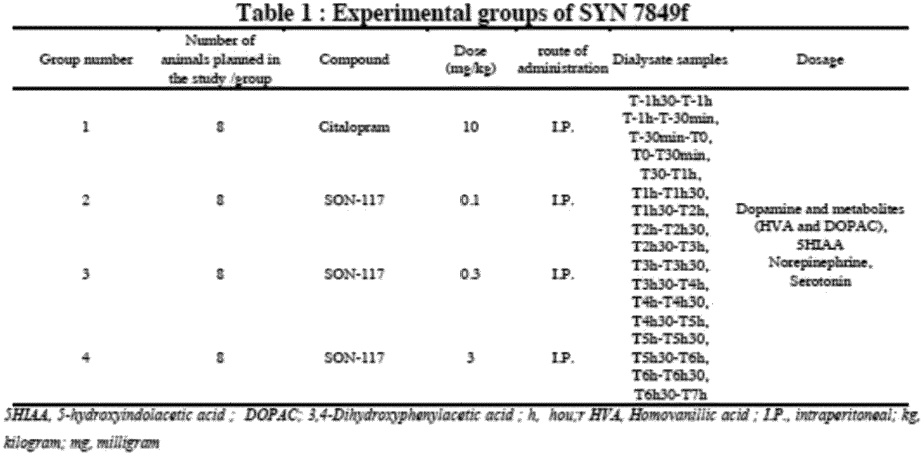

The experimental procedures were carried out in accordance to the European guidelines for the care and use of laboratory animals (Council Directive 86/609/EEC). Every effort was done to minimize animal suffering and reduce the number of animals used in the experiments. The acclimatization of the animals lasted at least 5 days. At receipt, animals were collectively housed in cages. Food and water was provided as a maintenance diet for animals (RM1, SDS Dietex), and was distributed daily in food and water dispensers.

Surgery was performed on rats weighing about 300 g at the beginning of the experiments. Rats were anaesthetized with Ketamine (50 mg/kg) & Xylazine (15 mg/kg) by intramuscular administration. The animals were placed in a stereotaxic apparatus in the flat-skull position at a constant setting of -3.3 mm between ear bars and the incisor bar. The animals were treated with a local anesthesic (xylocaine 1%, Astra Zeneca) and an analgesic (Temgesic, from Schering Plough, 0.05 mg/kg, s.c.). A rostrocaudal midline incision was made with a lancet to expose the dorsal skull surface. The "stereotaxic zero" was defined by the Bregma in accordance with Paxinos and Watson's atlas (1986). A hole was drilled in the skull with a drill and the meninges were pierced with a sterile needle to implant a CMA12 guide cannula which later received the dialysis probe.

Coordinates used for implanting guide cannula in prefrontal cortex from Bregma were anteroposterior 3.7 mm, mediolateral 0.8 mm, and dorsoventral -2mm (top of the probe was at - 5mm). The guide cannula was fixed to the skull with two stainless steel screws (1.88 mm outside diameter) anchored. An application of dental cement was added. A plastic collar allowed each animal to be tethered to a Swivel assembly of the CMA 120 awake animal system. Following surgery, rats were kept in individual cages and allowed to recover for a minimum of 3-5 days before the experimentation. The rats were handled and put in plastic bowl for 10 min at least every day to habituate the animals to their new environment for the dialysis. An I.P. administration with saline was also performed every day.

Dialysis probes (CMA12/3mm, membrane, 20 kD cut-off, Carnegie Medicine, Stockholm, Sweden), were carefully washed before inserting into the guide cannula. The wash was performed with the artificial cerebrospinal fluid (aCSF) medium (NaCl 147 mM, KC1 2.7 mM, CaCl₂ 1.2 mM, MgCl₂ 0.85 mM) at room temperature. The dialysis system was then equilibrated at 20°C at a perfusion rate of 1 µl/min with a Carnegie pump (CMA100). All catheters (FEP tubing, internal volume of 1.2 µl/10 cm, and tubing adaptors) used for microdialysis were treated in the same conditions.

Flow through the dialysis probe with aCSF solution was maintained at a constant rate of 1µl/min during the whole experimentation. The dialysis probe perfused with aCSF was placed in a vial containing the compound solution at 10⁻⁴ M for dopamine, DOPAC, 5-HIAA, norepinephrin and serotonin and at 10⁻³ M for HVA. The dialysate was collected from three different probes for 30 min in order to calculate a mean in vitro recovery for all the neurotransmitters tested. The collected fractions (30µl) were immediately transferred into dry ice and kept frozen (-80°C) until shipment. The percentage of in vitro recovery is the ratio between the concentration of the neurotransmitter that is recovered from the probe and the concentration in the mother solution.

The probes duly prepared were inserted into the guide cannula and the CMA120 Swivel assembly secured to the animal using a plastic collar. Flow through the dialysis probe with aCSF was maintained at a rate of 1µl/min during the whole experimentation. The first four fractions (corresponding to 2 hours) were discarded to avoid the effects of parenchymal disturbance and to ensure that an approximate steady-state level was reached. Fractions were then collected over a period of 8.5 h during which time the animals remained quiet. The dialysates were automatically collected with an autosampler (microfraction collector CMA 140; Carnegie Medicin) and stored at -80°C until analysis. In detail, after 1.5 hour of basal condition (three basal dialysates T-₁ₕ₃₀-T-₁ₕ, T-₁ₕ-T-₃₀ₘᵢₙ, T-₃₀ₘᵢₙ-T₀), animals were then administered I.P. with SON-117 or citalopram. Perfusion was then maintained for 7 hours with aCSF. The dialysates (30µl) were collected into 300µl vials, transferred into dry ice and then kept frozen (-80°C) until analysis. Frozen samples were shipped to a partner for analysis. At the end of the microdialysis study, a PBS with colored ink was perfused through the probe in order to visualize the dialyzed area.

The data generated in the micro-dialysis study provide strong evidence that SON-117 induces extracellular release of catecholamines, in particular 5-HT and DA, a finding that is consistent with the classic mechanism of action proposed for commonly-used mono-aminergic antidepressants. However, such neurotransmitter modulation in the micro-dialysis study is observed at doses that are much higher than doses that proved efficacious in the CMS model. For example, rapid-onset normalization of sucrose intake in the CMS model was observed at doses as low as 0.001 mg/kg, whereas the micro-dialysis study showed only a minor perturbation of neurotransmitter release at 0.3 mg/kg. This finding was unexpected and is supportive of a MoA for SON-117 to treat mood disorders that is distinct from other mono-aminergic antidepressants, for which the beneficial effect on mood is in the same dose range as the one inducing an extracellular release of catecholamines, in that it is not driven by catecholamine release and reuptake inhibition alone.

Catecholamine assay: Concentrations of dopamine, DOPAC, HVA, 5-HIAA, 5-HT, and norepinephrin were determined for each sample by HPLC coupled with electrochemical detection. The system consists of a pump (LC-10 AD; Shimadzu), a refrigerated automatic injector (Famos; Dionex), a reverse-phase Hypersil RP 18 column (Aquasil, 150 x 1 mm, 3 µm; ThermoHypersil), and an electrochemical detector (Decade, Antec) equipped with an amperometric cell (VT-03, Antec). Chromatograms were collected and treated with an integrator (CLAS VP, Shimadzu). The mobile phase consisted of sodium phosphate buffer (50 mM NaH₂PO₄), 1-octanesulfonic acid (1.7 mM), Na₂EDTA (200 µM) with 5% of acetonitrile at pH 3 for DA metabolites, and pH 4 for dopamine and Serotonin. The mobile phase was delivered by the pump at a flow rate of 60 µl/min⁻¹. The oxidation potential of the electrochemical cell was +650 mV. A volume of 6 µl was injected, and the running time for each determination was 35 min.

5-HIAA levels were also measured. Additional statistical analysis was performed for each experimental group and for each neurotransmitter or metabolites tested with a one-way repeated measure ANOVA.

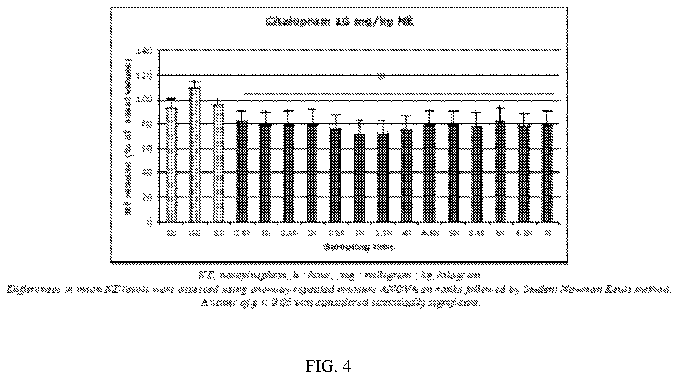

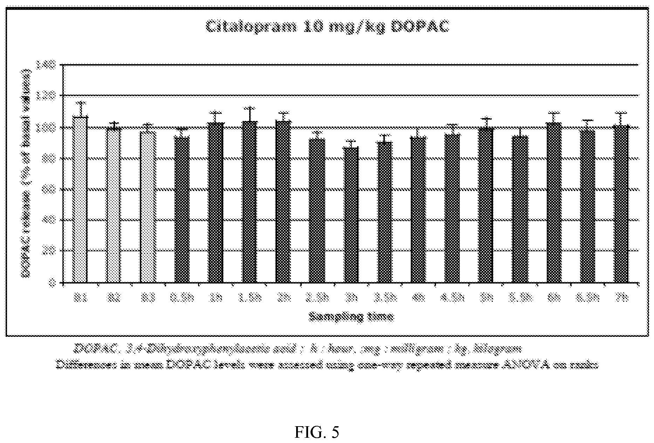

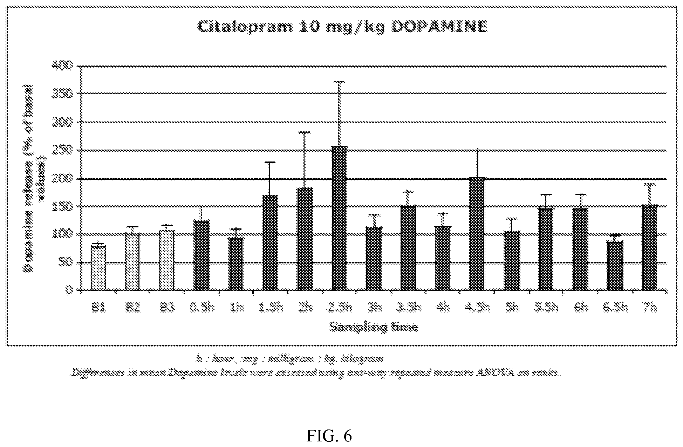

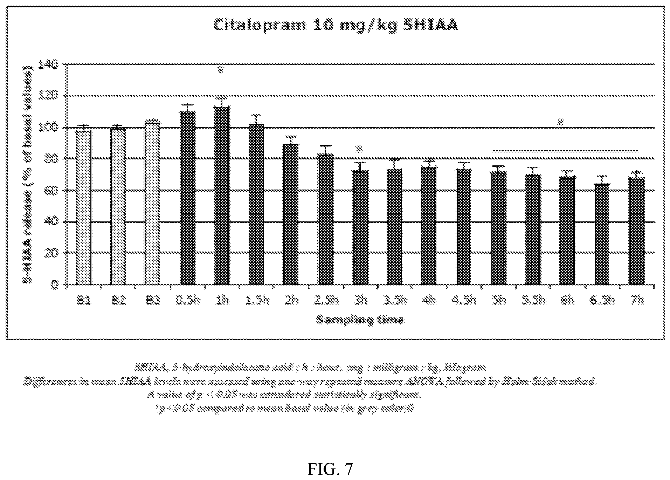

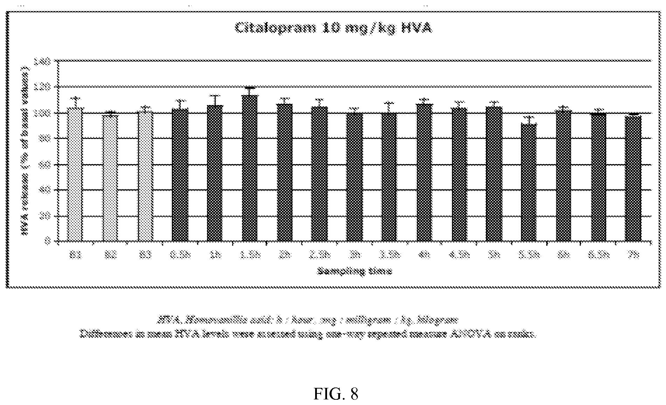

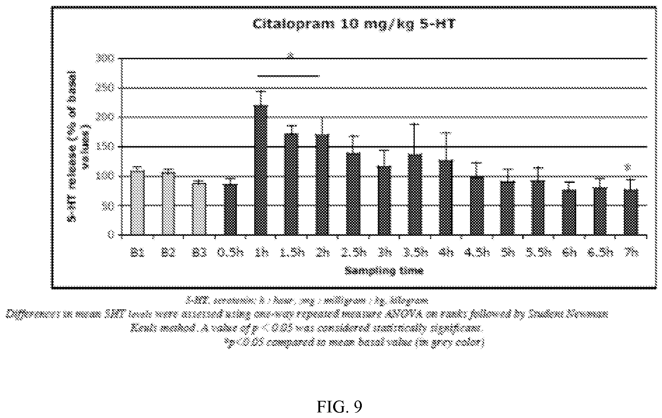

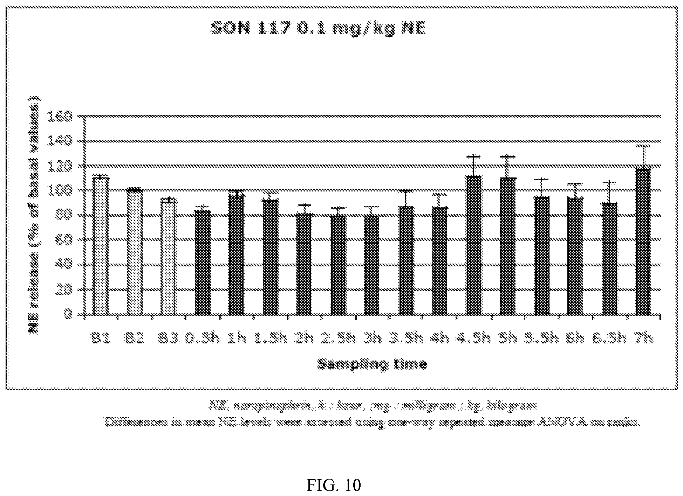

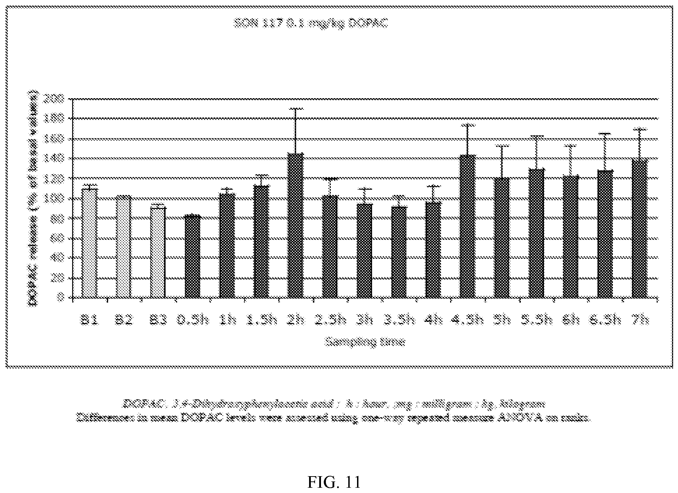

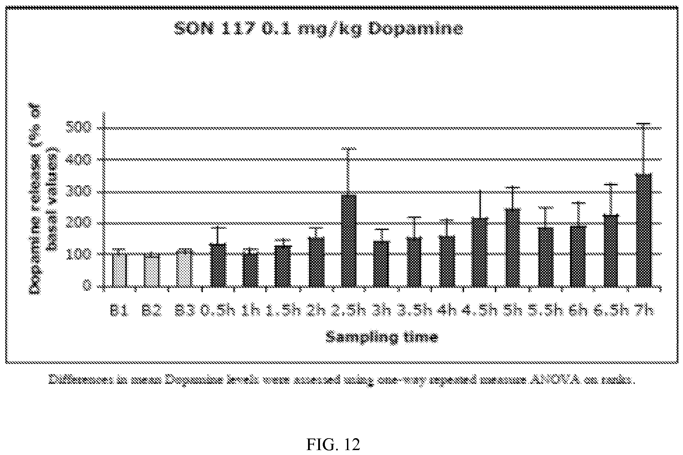

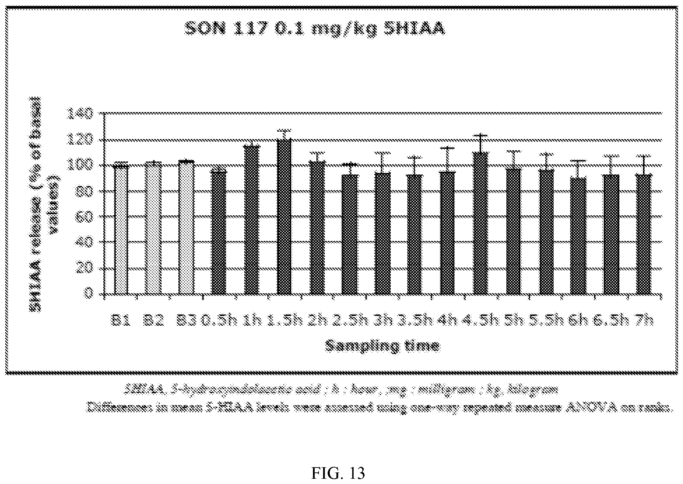

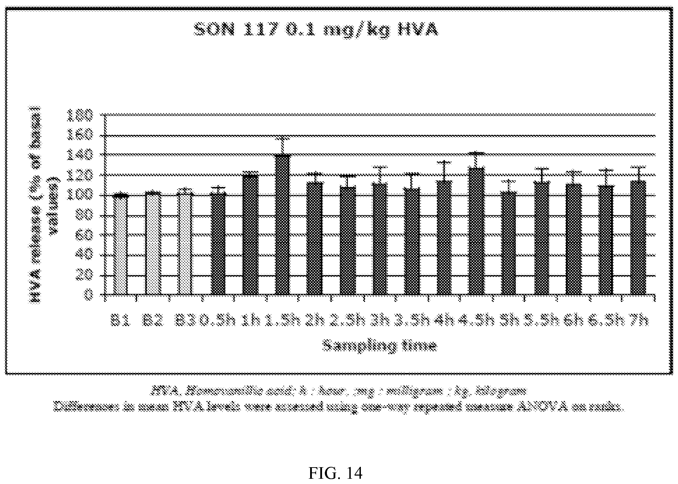

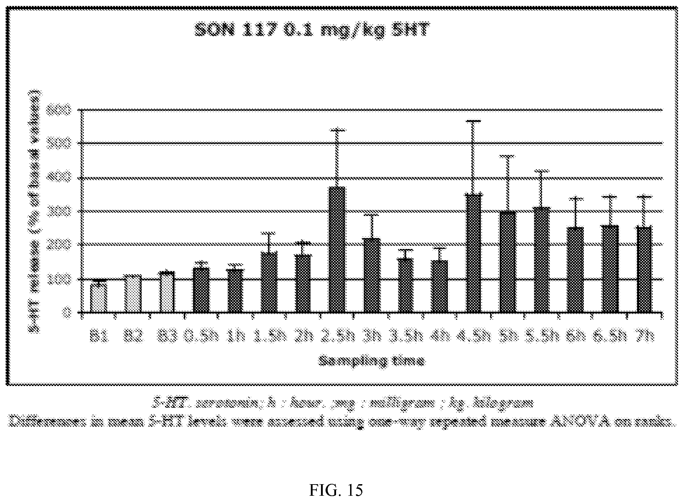

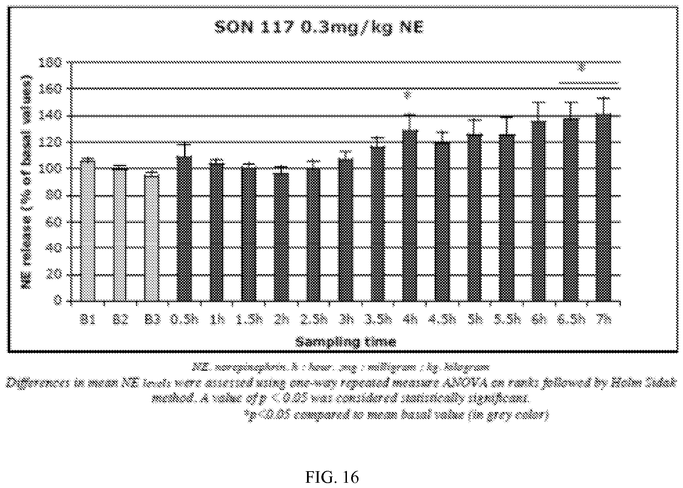

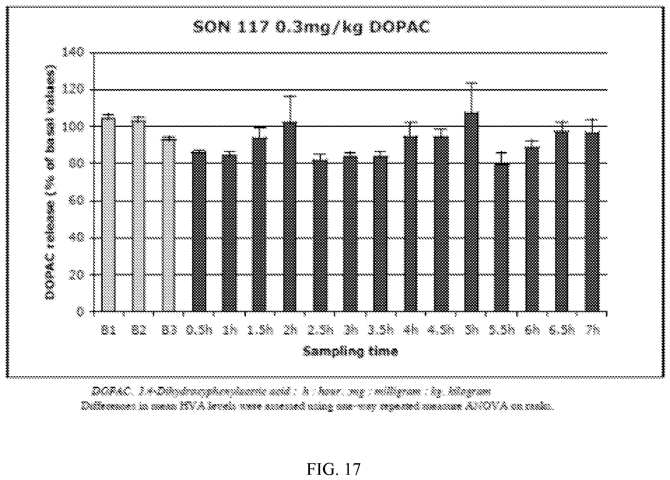

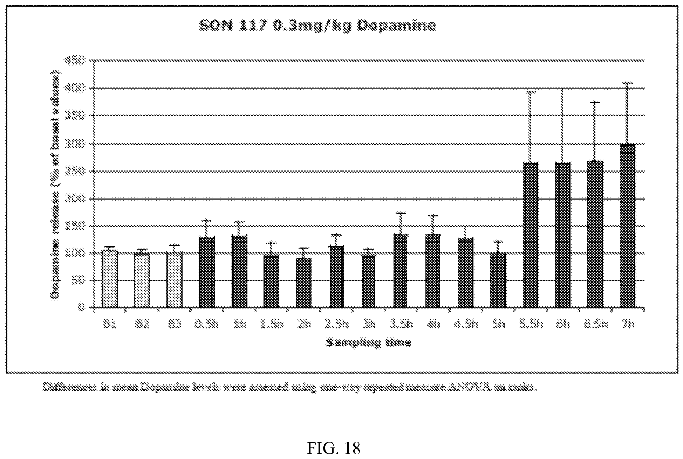

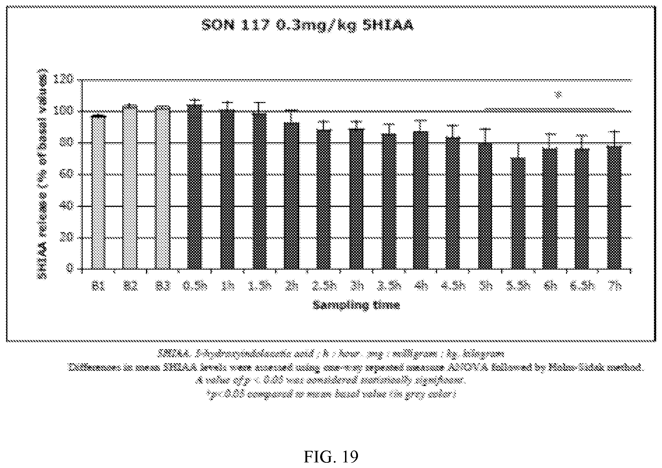

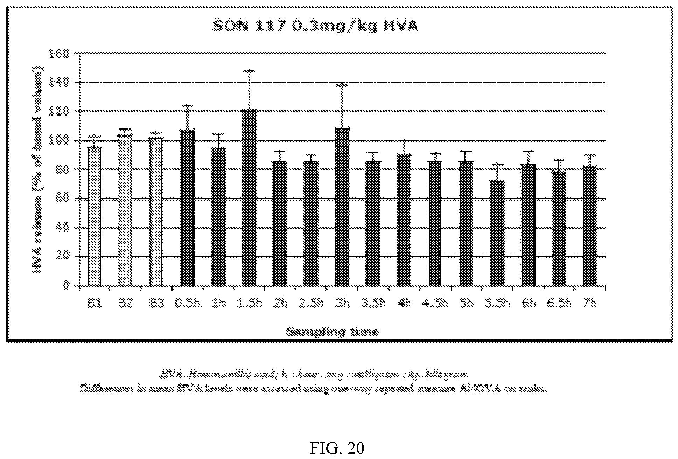

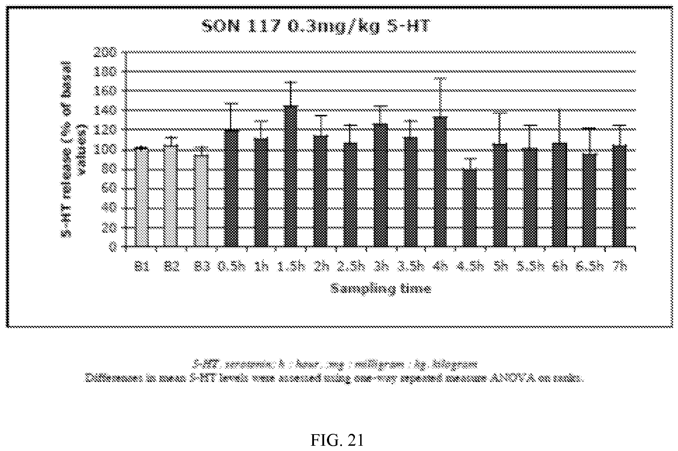

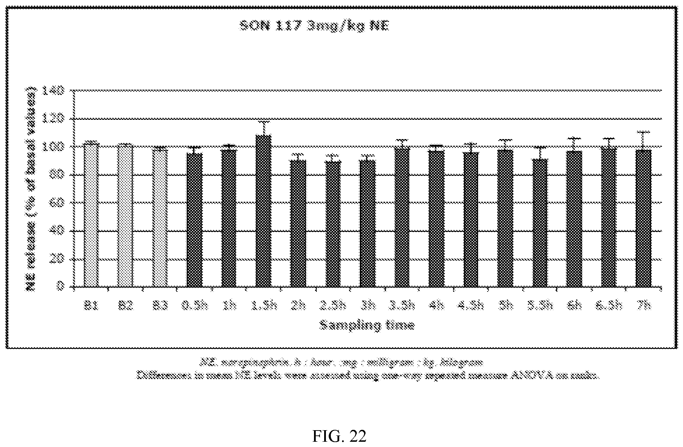

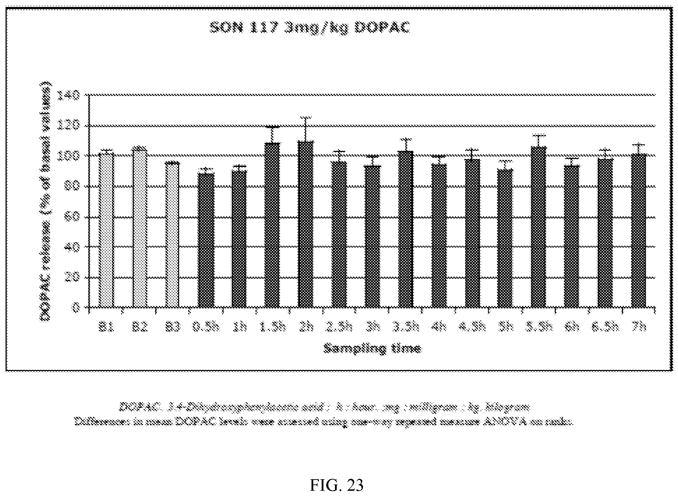

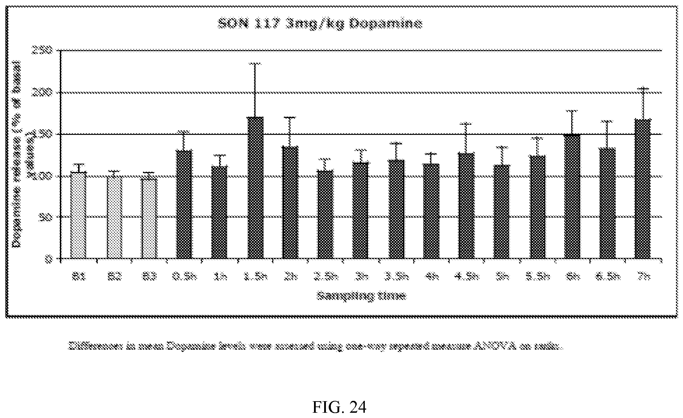

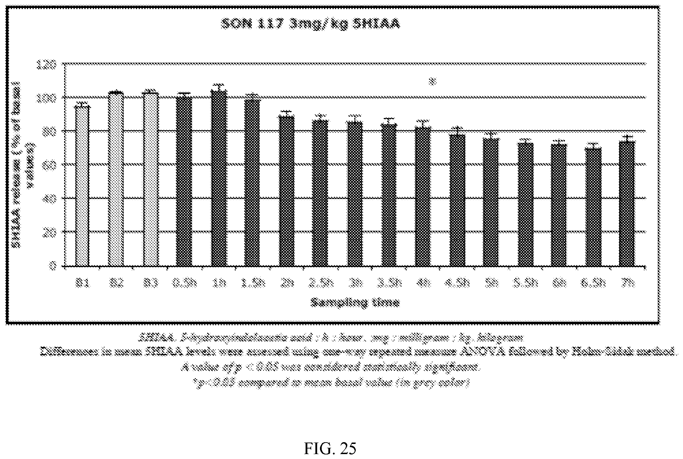

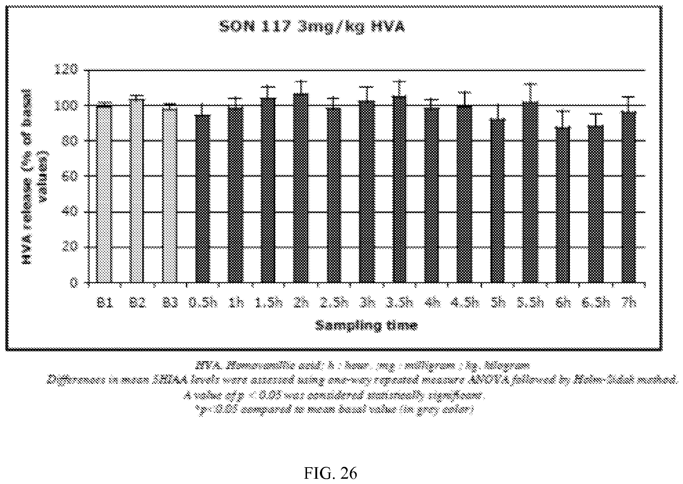

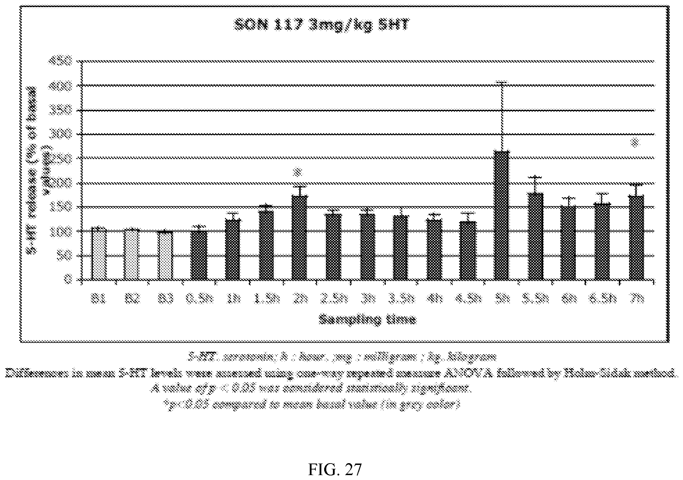

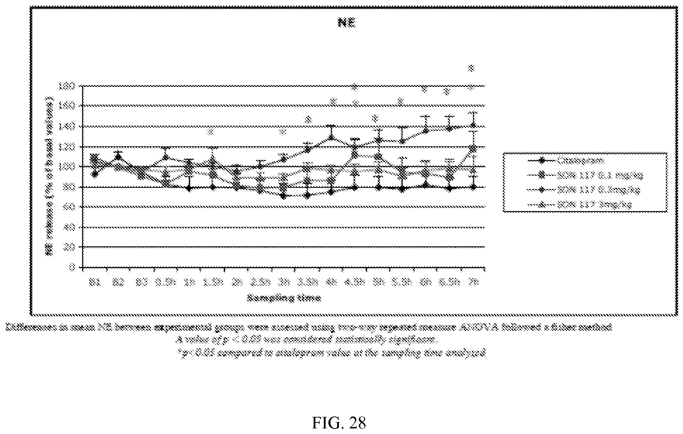

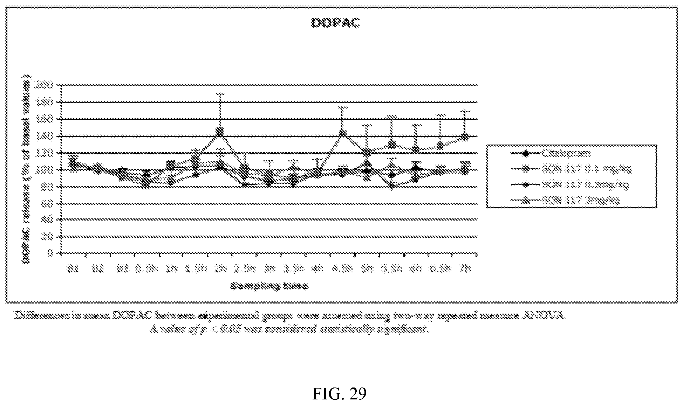

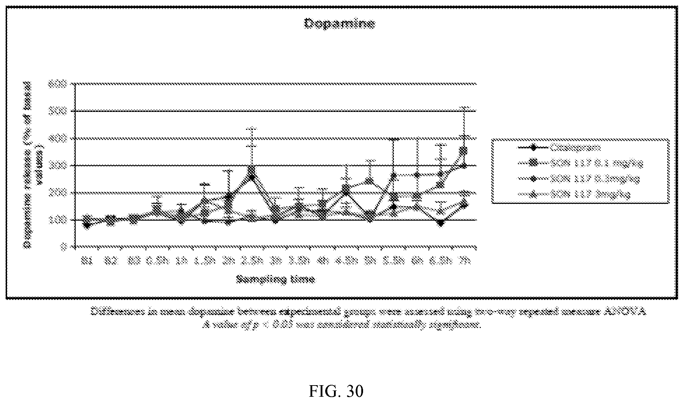

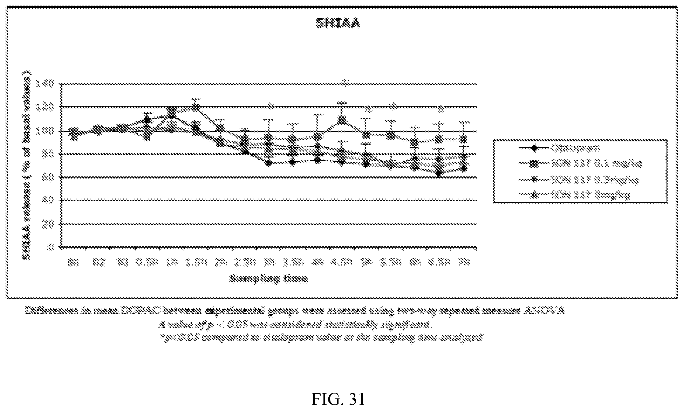

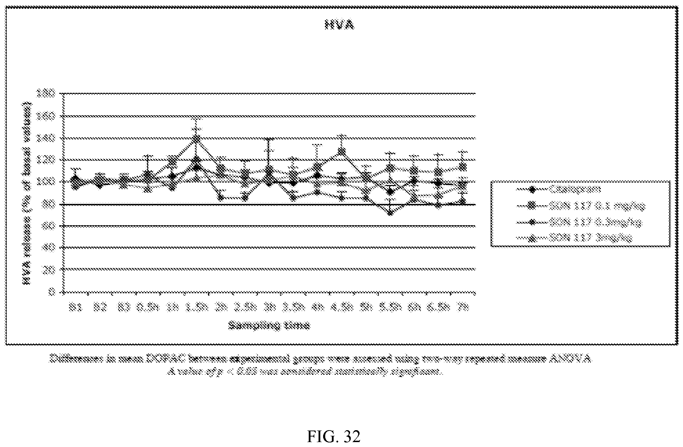

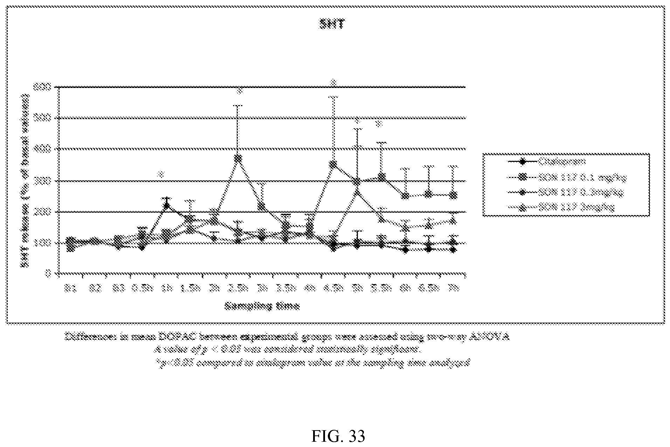

The following presentation of results is comprised of three sections. Table 2 presents the results obtained in the recovery tests. The tables and Figures 4 to 27 detail, for each treatment group, the individual and mean levels of each neurotransmitter or their metabolites measured in the dialysates samples before and after IP administration of the corresponding treatment. Finally, Figures 28 to 33 are the graphic representation of the cortical levels of each neurotransmitter or metabolites in the different experimental groups from TO to T7h.

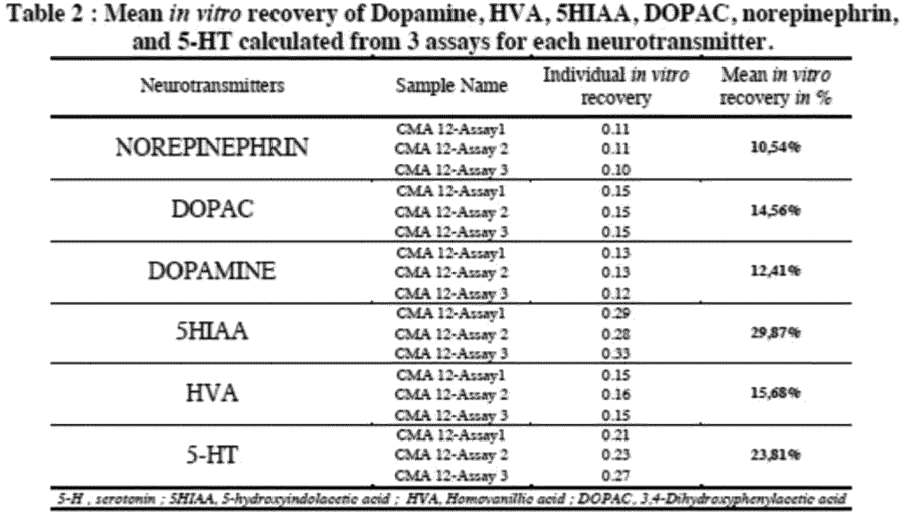

Samples collected during the in vitro recovery tests (Table 1) were analyzed for dopamine, HVA, DOPAC, norepinephrine, 5-HIAA and 5-HT concentration (measurements in dosing solutions and in dialysates). In Table 2, it is shown that the 3 independent assays for each neurotransmitter or metabolite are reproducible and a mean value of in vitro recovery for each neurotransmitter or metabolite can be calculated.

Results are expressed as mean ± SEM of the values obtained for the 8 animals of each experimental group and are expressed as percentage of the corresponding mean basal value for dopamine and metabolites (DOPAC, HVA), norepinephrine (NE), 5-HIAA and serotonin. 5-HIAA, a metabolite of 5-HT, was analyzed in the same HPLC run and was added to the results.

Tables 3, 4, 5, 6, 7 and 8 present the concentrations of norepinephrine, DOPAC, dopamine, 5-HIAA, HVA and 5-HT in dialysates (in % of respective basal value) in the citalopram 10 mg/kg, I.P treated group.

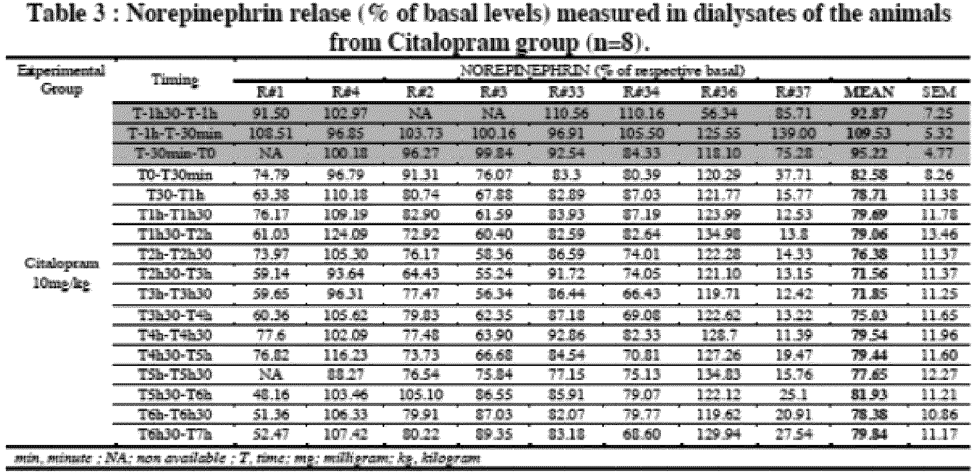

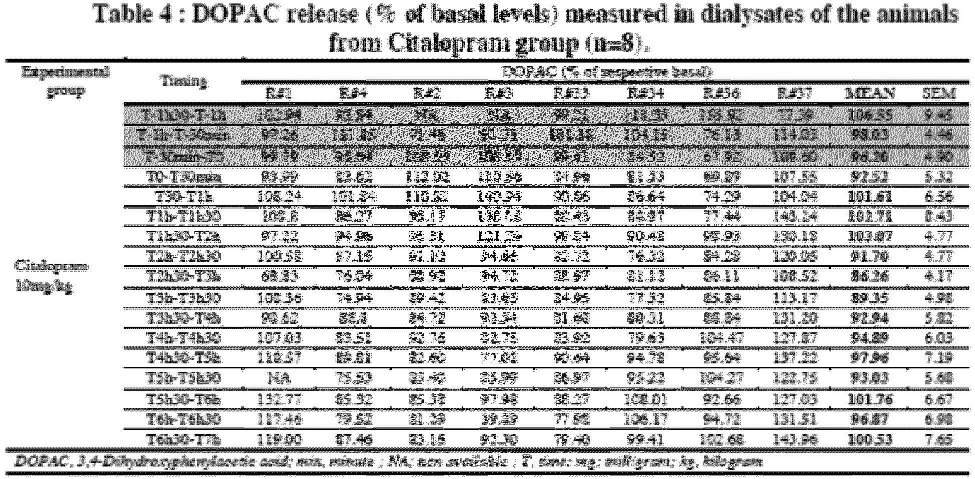

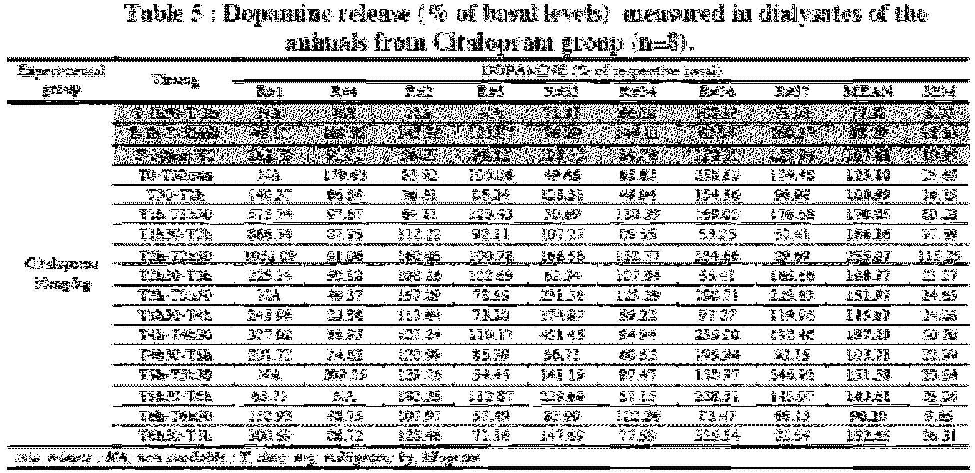

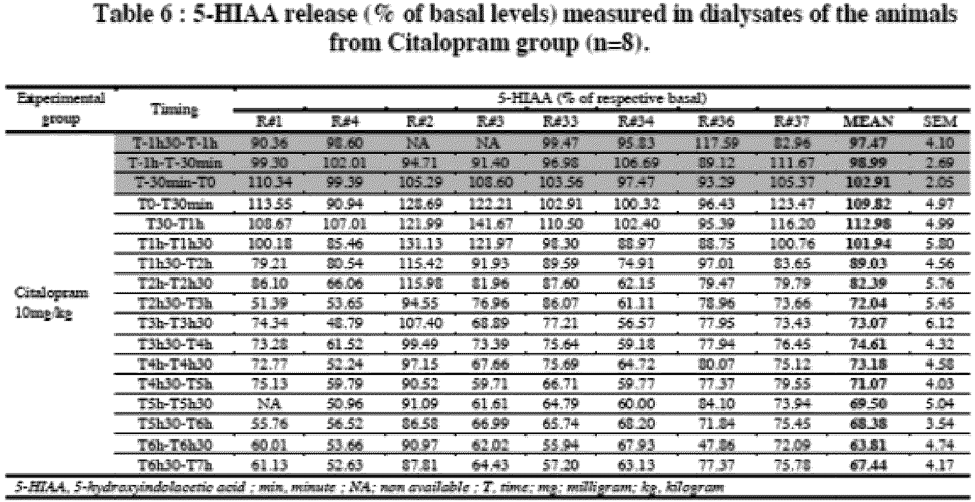

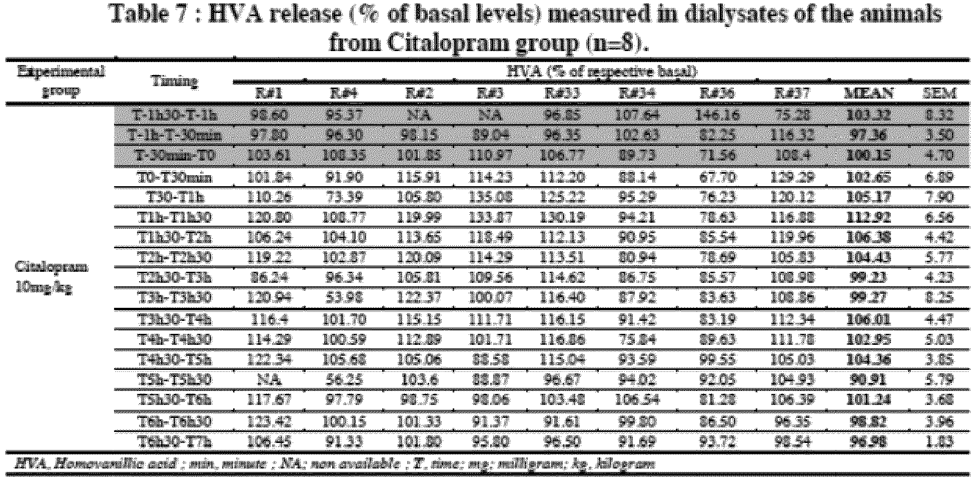

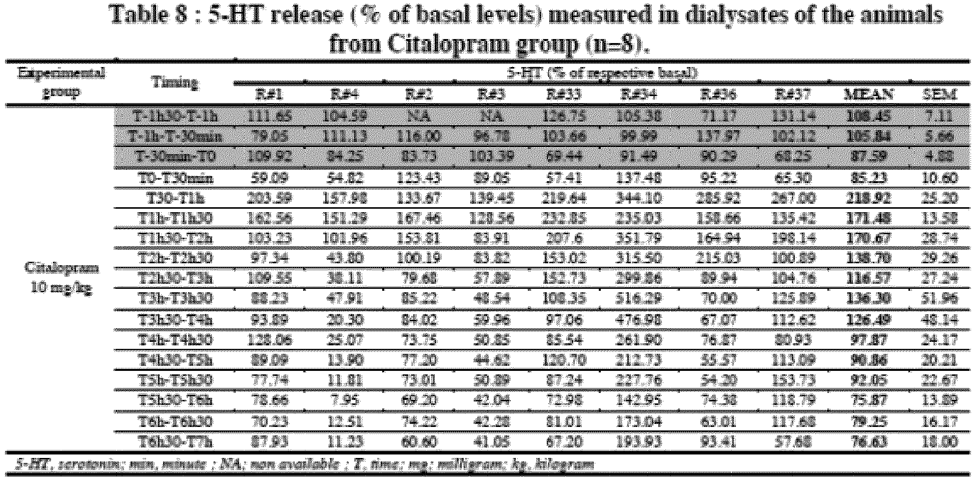

The I.P. administration of citalopram induced a significant and stable slight decrease in NE concentration (72%) in prefrontal cortex from 0.5h to 7h after administration. For DOPAC, no statistically significant differences were observed after administration of citalopram (10mg/kg). The I.P. administration of citalopram (10mg/kg) tended to induce an increase in dopamine levels from T1.5h and 2.5h (255%). This increase was not significant. The IP administration of citalopram (10mg/kg) induced a significant slight increase in 5-HIAA concentration in prefrontal cortex at 0.5h and 1h (113%) after its administration followed by a significant and stable decrease from 2h to 7h after its administration. For HVA, no statistically significant differences were found after administration of citalopram (10mg/kg). The IP administration of citalopram (10mg/kg) induced a significant increase in 5-HT concentrations (219%) in prefrontal cortex from 1h to 2h followed by a significant slight decrease in 5-HT levels from 6h to 7h after its administration.

Tables 9, 10, 11, 12, 13 and 14 present the concentrations of norepinephrin, DOPAC, dopamine, 5-HIAA, HVA and 5-HT in dialysates (in % of their respective basal value) in the SON-117 0.1 mg/kg, I.P. treated group. For NE, no statistically significant differences were found between the mean pre-injection value and the post injection levels of SON-117 (0.1 mg/kg). The I.P. administration of SON-117 (0.1 mg/kg) tended to induce a slight increase in DOPAC levels from 1.5h to 2.5h and from 4.5h to 7h. This increase was not statistically significant. The I.P. administration of SON-117 (0.1mg/kg) tended to induce an increase in dopamine levels (287%) from T1.5h and 2.5h and from 4.5h to 7h (353%). This increase was not statistically significant. For 5-HIAA, no statistically significant differences were found between the mean pre-administration value and the dialysate levels after the I.P. administration of SON-117 (0.1 mg/kg). For HVA, no statistically significant differences were found between the mean pre-administration value and the dialysate levels after the I.P. administration of SON-117 (0.1 mg/kg). The IP administration of SON-117 (0.1mg/kg) tended to induce an increase in serotonin levels at 2.5h (368%) and from 4.5h to 7h (350%). This increase was not; statistically significant.

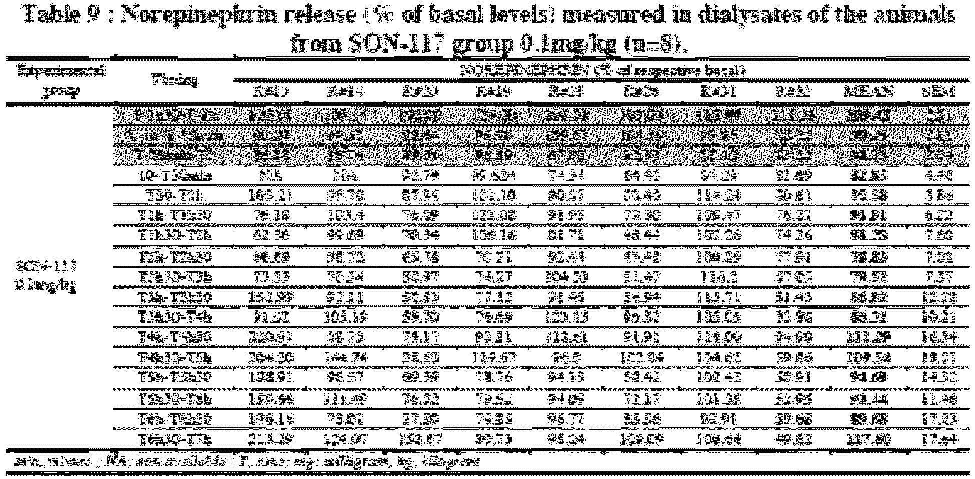

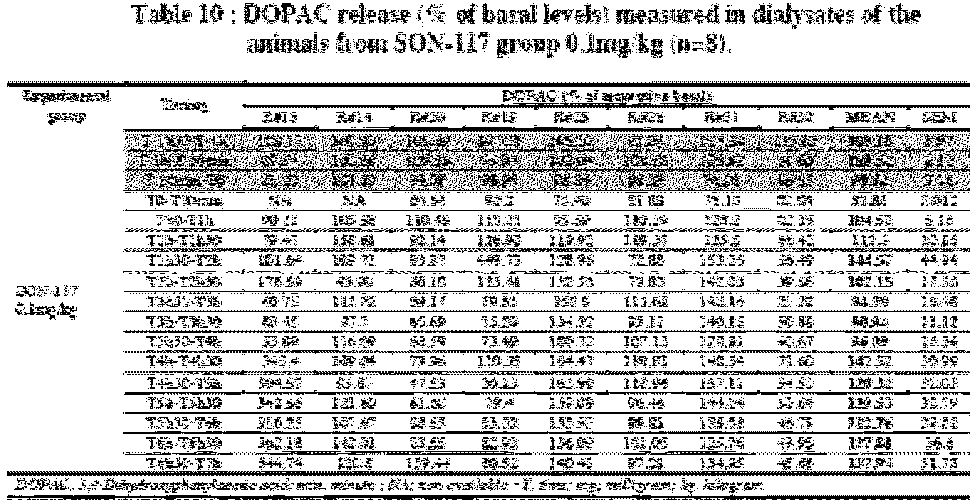

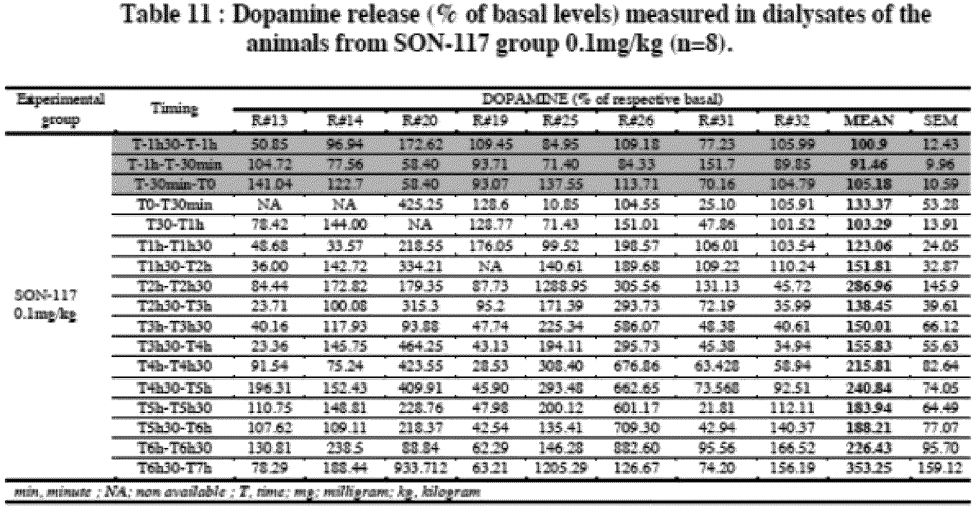

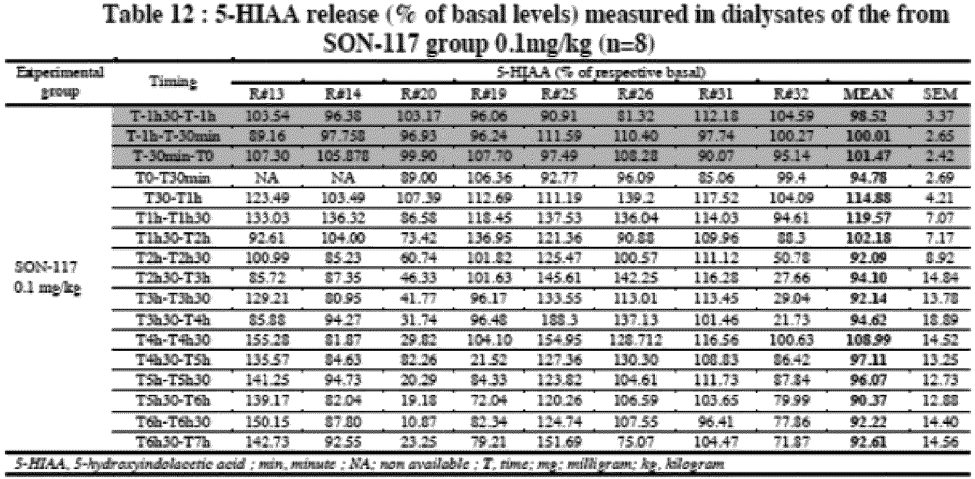

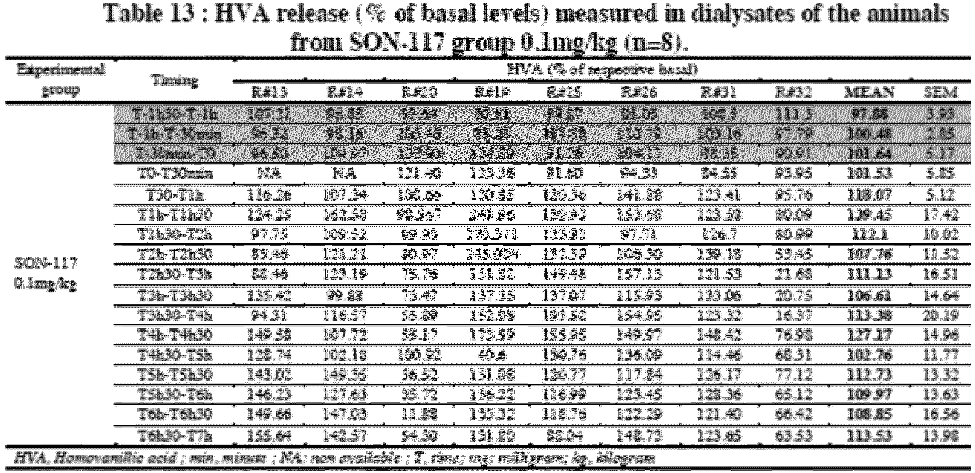

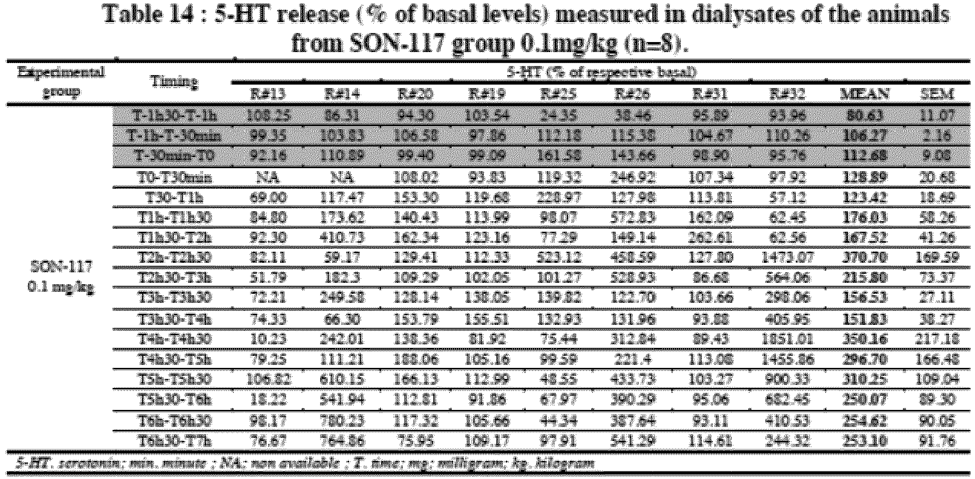

Tables 15, 16, 17, 18, 19 and 20 present the concentrations of norepinephrine, DOPAC, dopamine, 5-HIAA, HVA and 5-HT in dialysates (in % of respective basal value) in the SON-117 0.3 mg/kg, I.P. treated group. The I.P. administration of SON-117 (0.3mg/kg) induced a significant slight increase in NE concentrations in prefrontal cortex at 3.5h (117%) and from 4.5h to 7h (128%) after its administration. For DOPAC, no statistically significant differences were found between the mean pre-administration value and the dialysate levels after the IP administration of SON-117 (0.3 mg/kg). The I.P. administration of SON-117 (0.3mg/kg) tended to induce an increase in dopamine levels from 5.5h to 7h (298%). This increase was not statistically significant. The IP administration of SON-117 (0.3mg/kg) induced a significant and stable slight decrease in 5-HIAA concentrations in prefrontal cortex at 3.5h and from 4.5h to 7h after its administration. For HVA, no statistically significant differences were found between the mean pre-administration value and the dialysate levels after the IP administration of SON-117 (0.3 mg/kg). For 5-HT, no statistically significant differences were found between the mean pre-administration value and the dialysate levels after the IP administration of SON-117 (0.3 mg/kg).

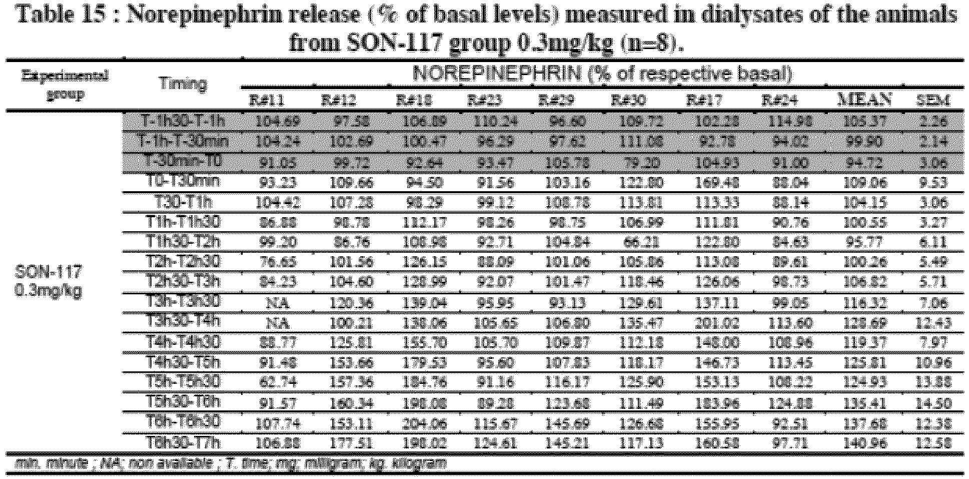

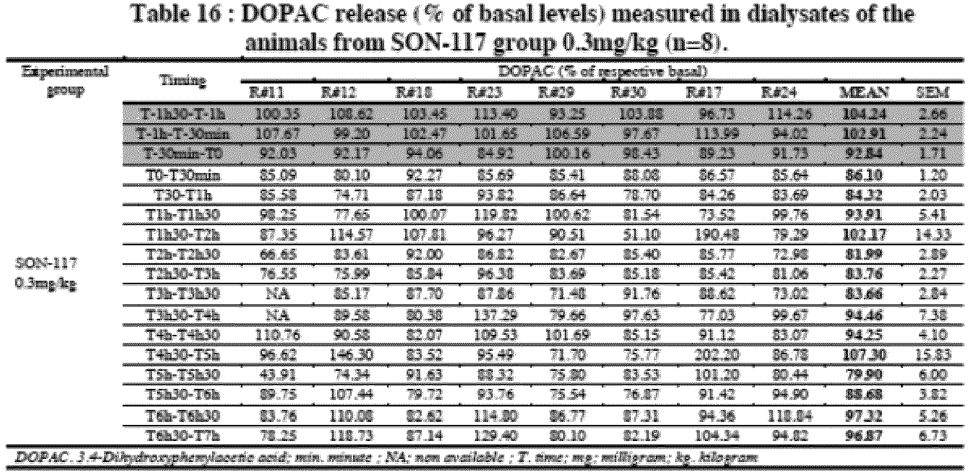

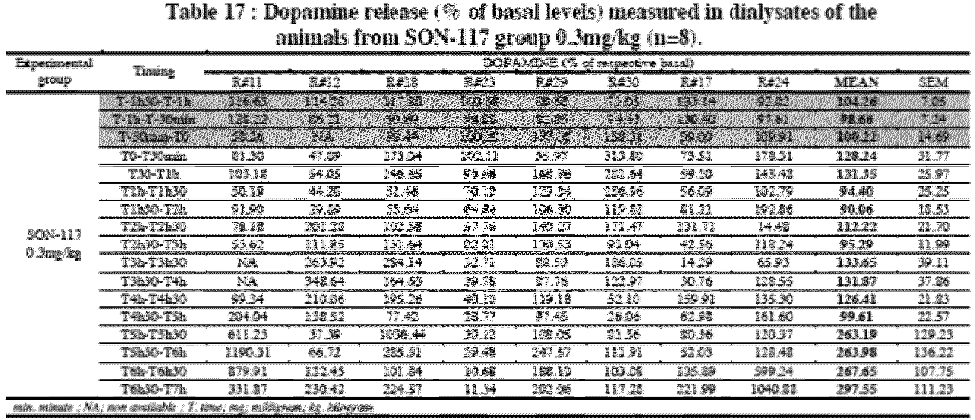

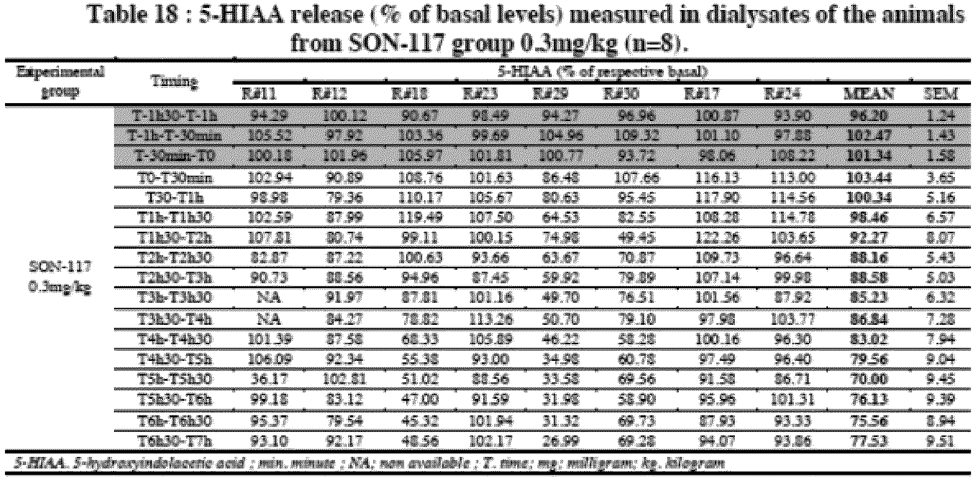

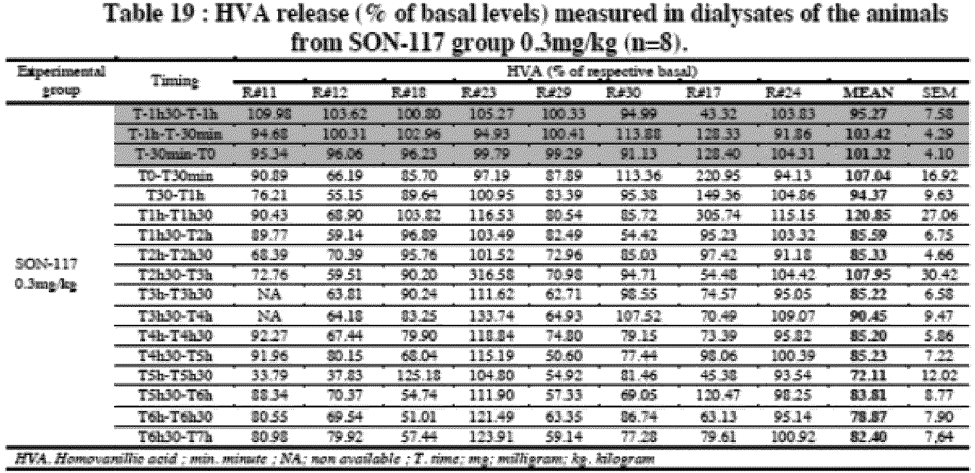

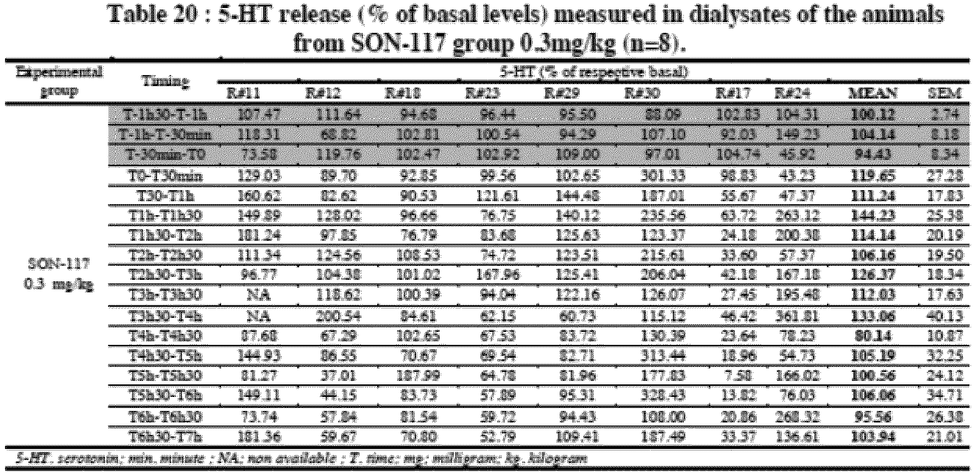

Tables 21, 22, 23, 24, 25 and 26 present the concentrations of norepinephrine, DOPAC, dopamine, 5-HIAA, HVA and 5-HT in dialysates (in % of respective basal value) in the SON-117 3 mg/kg IP treated group. For NE, no statistically significant differences were found between the mean pre-administration value and the dialysate levels after the IP administration of SON-117 (3 mg/kg). For DOPAC, no statistically significant differences were found between the mean pre-administration value and the dialysate levels after the IP administration of SON-117 (3 mg/kg). The IP administration of SON-117 (3 mg/kg) tended to induce a slight increase in dopamine levels at 1.5h (169%) and from 6h to 7h (max. 167%); This increase was not statistically significant. The IP administration of SON-117 (3 mg/kg) induced a significant and stable decrease in 5-HIAA concentration in prefrontal cortex from 2h to 7h (max. 72%) after its administration. The IP administration of SON-117 (3mg/kg) induced a significant slight increase in HVA concentration in prefrontal cortex at 3.5h (114%) after its administration. The IP administration of SON-117 (3 mg/kg) induced a significant increase in 5-HT concentrations in prefrontal cortex from 2h to 3h (max. 175%) and from 5.5h to 7h (272%) after its administration.

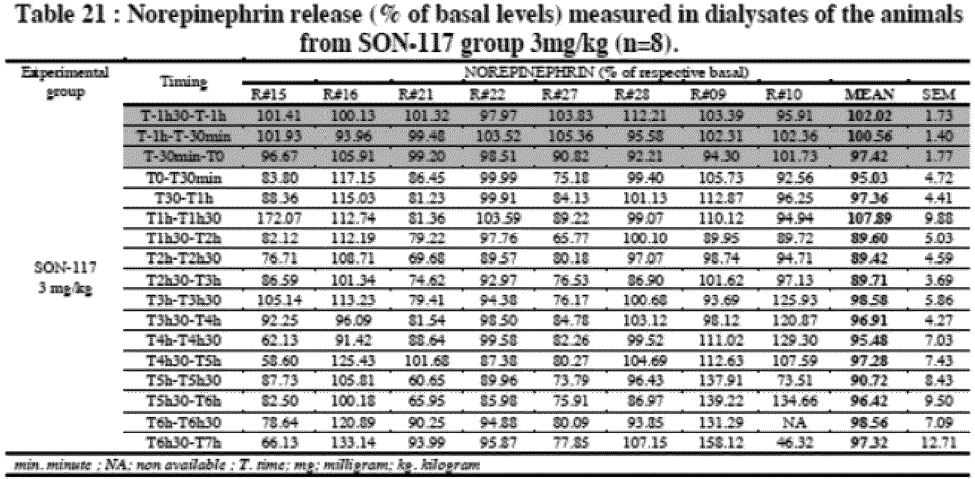

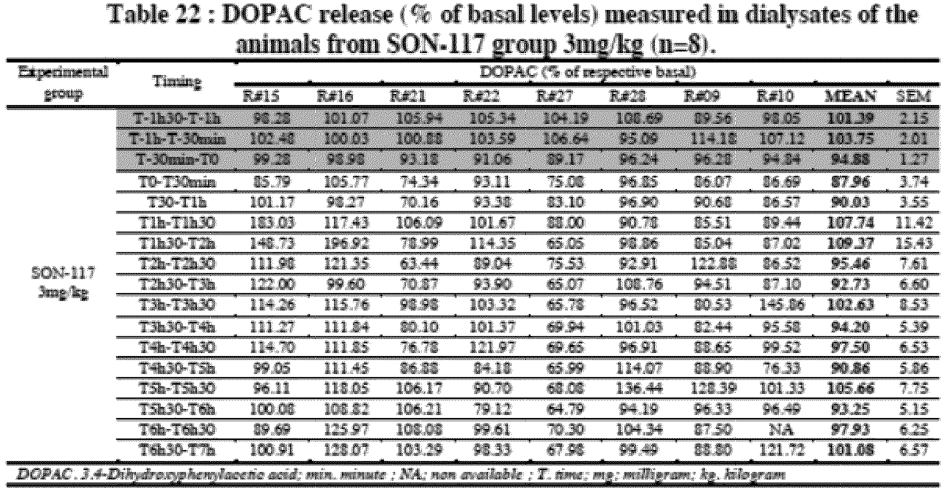

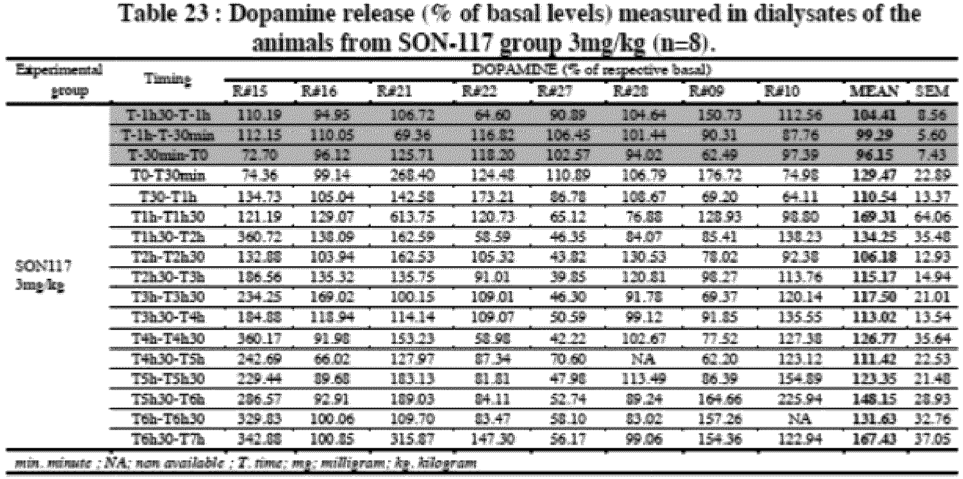

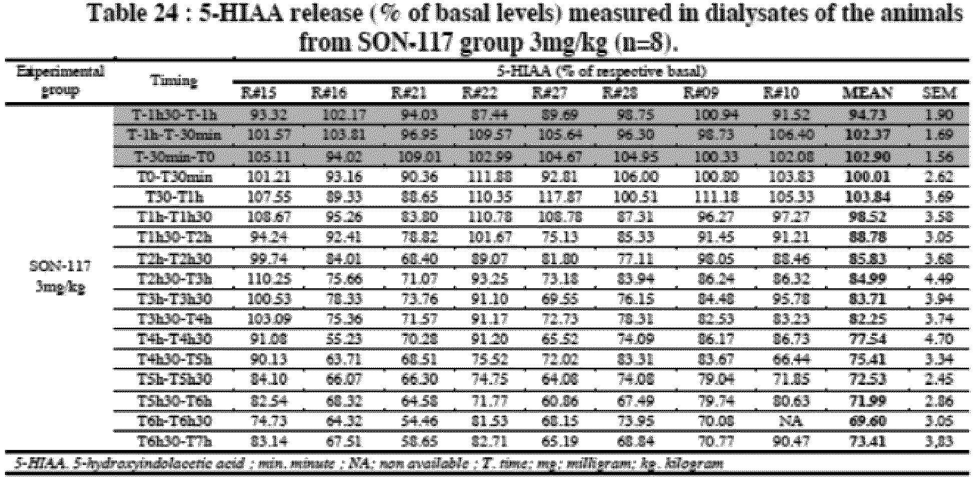

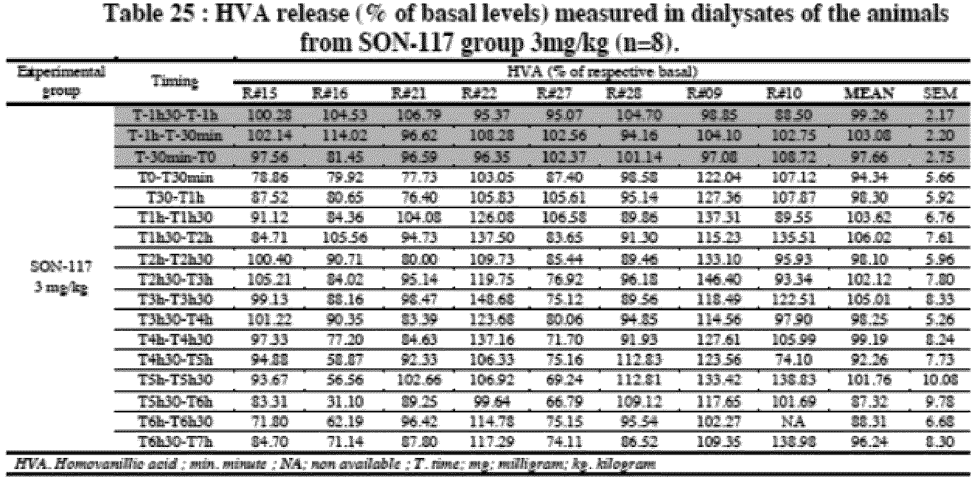

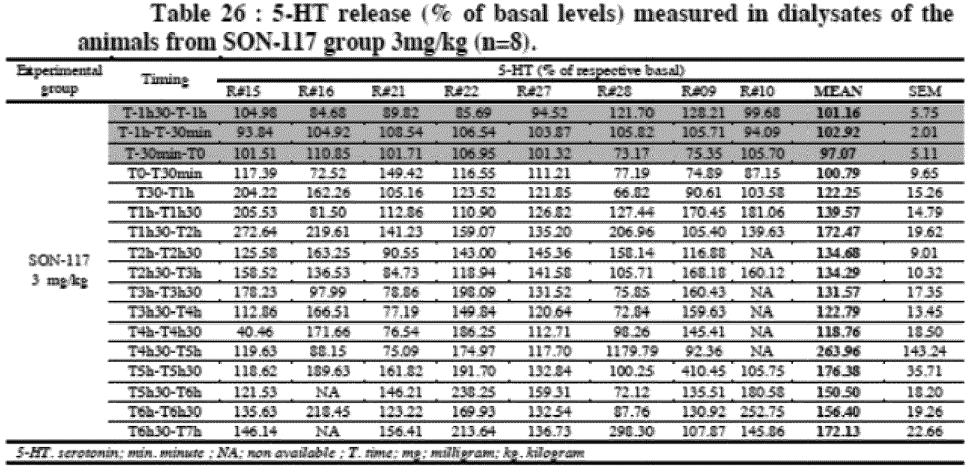

A global analysis was performed to compare the effect of SON-117, administered I.P. at the three concentrations (0.1, 0.3 and 1 mg/kg), on the catecholamines release in the rat prefrontal cortex vs. citalopram administered IP at 10 mg/kg.

For NE, the global two-way ANOVA analysis revealed that: (1) There is a significant difference between the group treated with citalopram and SON-117 at 0.1mg/kg at the sampling time 4.5h and 7h. At 4.5h and 7h, after compound administration, the levels of NE were higher in the SON-117 (0.1 mg/kg) group compared to the citalopram treated group; (2) There is a significant difference between the group treated with citalopram and SON-117 at 0.3mg/kg at the sampling time 3.5h, 4h, 4.5h, 5h, 5.5h, 6h and 7h. At these sampling time points after compound administration, the levels of NE were higher in the SON-117 (0.3 mg/kg) group compared to the citalopram treated group; and (3) There is a significant difference between the group treated with citalopram and SON-117 at 3mg/kg at the sampling time 1.5h and 3.5h. At these two sampling time points after compound administration, the levels of NE were higher in the SON-117 (3 mg/kg) group compared to the citalopram treated group.

For DOPAC, the global two-way ANOVA analysis revealed that, there is no significant differences between the group treated with citalopram and SON-117 at the three doses tested in function of time. However we observe a trend for an increased release of DOPAC with SON-117 0.1 mg/kg at 2h and from 4.5h to 7h.

For dopamine, the global two-way ANOVA analysis revealed that, there is no significant differences between the group treated with citalopram and SON-117 at the three doses tested in function of time. However we observe a trend for an increased release at two doses of SON-117 (0.1 and 0.3 mg/kg) from 5.5h.

For 5-HIAA, the global two-way ANOVA analysis revealed that: (1) There is a significant difference between the group treated with citalopram and SON-117 at 0.1mg/kg at the sampling time 0.5h, 4.5, 5h and 6.5h. At 0.5h after compound administration, the levels of 5-HIAA were lower in the SON-117 group (0.1 mg/kg) group compared to the citalopram treated group. Conversely, at 4.5h, 5h, 6.5h after compound administration, the levels of 5-HIAA were higher in the SON-117 group (0.1 mg/kg) group compared to the citalopram treated group; (2) There is no significant difference between the group treated with citalopram and SON-117 at 0.3 mg/kg in function of time; and (3) There is a significant difference between the group treated with citalopram and SON-117 at 3 mg/kg at the sampling time 3h and 3.5h. At these two time points after compound administration, the levels of 5-HIAA were higher in the SON-117 group (3 mg/kg) group compared to the citalopram treated group.

For HVA, the global two-way ANOVA analysis revealed that, there is no significant differences between the group treated with citalopram and SON-117 at the three doses tested in function of time.

For 5-HT, the global two-way ANOVA analysis revealed that: (1) There is a significant difference between the group treated with citalopram and SON-117 at 0.1mg/kg at the sampling time 2.5h and 4.5 and 5.5h. A trend for a continuous increased release of dopamine is observed from 4.5h to 7h. At these sampling time points after compound administration, the levels of 5-HT were higher in the SON-117 group (0.1 mg/kg) group compared to the citalopram treated group. The maximum increase observed with SON-117 0.1 mg/kg at 2.5h is 369% compared with a maximum increase of 218% with citalopram at 1h. From 4.5h to 7h an elevated increase release of 5-HT is maintained at a level comprised between 349% and 249%; (2) There is a significant difference between the group treated with citalopram and SON-117 at 0.3 mg/kg at the sampling time 1h. The levels of 5-HT were slightly lower in the SON-117 group compared to the citalopram treated group; and (3) There is a significant difference between the group treated with citalopram and SON-117 at 3mg/kg at the sampling time 1h and 5h and 7h. At 1h after compound administration, the levels of 5-HT were slightly lower in the SON-117 group (3 mg/kg) group compared to the citalopram treated group. Conversely, at 5h and 7h after compound administration, the levels of 5-HT were higher in the SON-117 group (3 mg/kg) group compared to the citalopram treated group. A trend for a continuous increased release of dopamine is observed from 5h. The maximum increase observed at 5h is 272% (superior to the maximum increase of 218% observed with citalopram). An increase of 183% was still present 7h after SON-117 administration.

The aim of the experiment was to, in part, assess variations of NE, dopamine and its metabolites (DOPAC and HVA) and serotonin and its metabolite 5-HIAA in the prefrontal cortex of rats after an IP administration of SON-117 at three doses (0.1, 0.3 and 3 mg/kg). In part, the aim of the project was also to compare these variations induced by SON-117 to the effects of the reference compound, citalopram.

The present study showed that the administration of citalopram (10mg/kg; IP) was first able to induce an increase in the 5-HT levels in the prefrontal cortex between 1 and 2h and a decrease in 5-HT levels between 6 and 7h after its administration. These variations of 5-HT were associated with those of 5-HIAA where a first transient increase in 5-HIAA levels was observed followed by a decrease in the concentration of the metabolite. Regarding dopamine, the administration of citalopram did not affect the neurotransmitter levels and its metabolites DOPAC, HVA in the prefrontal cortex of rat. For dopamine, the heterogeneity of the response between the animals of the citalopram group might explain why the increase in dopamine levels observed after the administration of citalopram was not statistically significant. Finally, citalopram (10mg/kg IP) induced a significant and stable slight decrease in NE levels measured after its IP administration.

SON-117 at the three doses tested induced an increased release of NE at different sampling times. The increase was more pronounced from 3.5h after the administration. However, the increase remained moderate with a maximum of 129% 7h after administration. For dopamine, a trend for an increased release appeared at 0.1 and 0.3 mg/kg at late times after administration (from 5h at 0.1 mg/kg and from 5.5h at 0.3 mg/kg). At the two doses of 0.1 and 3 mg/kg, SON-117 significantly increased the release of 5-HT. Interestingly, the increases were superior to those observed with citalopram (369% vs. 218% for the maximum observed). Moreover, the increases observed with SON-117 were maintained from 4.5h to 7h. Regarding the metabolites of dopamine (DOPAC and HVA) and the metabolite of 5-HT (5-HIAA), the effects were less pronounced. Nevertheless, it was noted that the effects of SON-117 at 0.1 mg/kg on DOPAC release were very close to those observed with dopamine (an increase at 2h and from 4h to 7h), and the levels of 5-HIAA were also modified at late times after administration of SON-117 0.1 mg/kg.

This study is the first study to explore the effects of SON-117 after IP administration on the release of dopamine, 5-HT, norepinephrine and the metabolites DOPAC, HVA and 5-HIAA. A large time-frame was covered, from TO to 7h after administration, and showed surprising effects of SON-117 mainly on dopamine and 5-HT, these effects being superior to those observed with citalopram. Moreover, the effects of SON-117 appeared to be sustained in the time, suggesting a role for the activity of SON-117 at times beyond 7h after administration.

### Example 4: Chronic Stress Model in Rats Using SON-117

Male Wistar rats (Charles River, Germany) were brought into the laboratory one month before the start of the experiment. Except as described below, the animals were singly housed with food and water freely available, and were maintained on a 12-h light/dark cycle, in a constant temperature (22 ± 2°C) and humidity (50 ± 5%) conditions.

After a period of 2 weeks of adaptation to laboratory and housing conditions, the animals were first trained to consume a 1% sucrose solution; training consisted of nine 1h baseline tests in which sucrose was presented, in the home cage, following 14h food and water deprivation; the sucrose intake was measured by weighing pre-weighed bottles containing the sucrose solution, at the end of the test. Subsequently, the sucrose consumption was monitored, under similar conditions, at weekly intervals throughout the whole experiment.

On the basis of their sucrose intakes in the final baseline test, the animals were divided into two matched groups. One group of animals was subjected to the chronic mild stress procedure for a period of 8 consecutive weeks. Each week of stress regime consisted of: two periods of food or water deprivation, two periods of 45 degree cage tilt, two periods of intermittent illumination (lights on and off every 2h), two periods of soiled cage (250 ml water in sawdust bedding), one period of paired housing, two periods of low intensity stroboscopic illumination (150 flashes/min), and three periods of no stress. All stressors were 10 - 14 h of duration and were applied individually and continuously, day and night. Control animals were housed in separate rooms and had no contact with the stressed animals. They were deprived of food and water for the 14h preceding each sucrose test, but otherwise food and water were freely available in the home cage.

On the basis of their sucrose intakes following initial 2 weeks of stress, both the stressed and the control groups were each divided further into matched subgroups (n = 8), and for subsequent five weeks they received once daily intraperitoneal injections of vehicle (0.5% hydroxypropylmethylcellulose, 1 ml/kg), SON-117 (0.001, 0.01, 0.1 and 1 mg/kg) or imipramine (10 mg/kg) as the reference treatment. The drugs were administered at approx. 10:00 AM and the weekly sucrose tests were carried out 24h following the last drug injections. After five weeks all treatments were terminated and one additional sucrose test was carried out after one week of withdrawal. Stress was continued throughout the period of treatment and withdrawal.

After completion of the administration and withdrawal period, all control and stressed animals were placed in an open field (100 cm in diameter, 35 cm high) and allowed to explore two identical objects (cylinder-shaped object with walls painted white, 7 cm in diameter, 11 cm high) for the time required to complete 15 s of exploration of either object. For the retention trial conducted one hour later, one of the objects presented previously was replaced with a novel object (prism-shaped object with walls painted black, 5 cm wide, 14 cm high). Rats were returned to the open field for 5 min and the duration of exploration (i.e. sitting in close proximity to the objects, sniffing or touching them) of each object was measured separately by a trained observer. The data were calculated according to the following formula: [time of novel object exploration divided by time of novel plus familiar object exploration multiplied by 100]. The CMS and NOR data were analyzed by multiple analyses of variance with three between-subjects factors (stress/control, drug treatments and intakes in successive sucrose tests). The Fisher's LSD test was used for the post-hoc comparisons of means.

The results of the study are as follows. Chronic mild stress caused a gradual decrease in the consumption of 1% sucrose solution. In the final baseline test all animals drank approximately 13 g of sucrose solution. Following initial two weeks of stress the intakes remained at similar level in controls but fell to approximately 8 g in stressed animals [F(1,94) = 169.289; p<0.001], and such a difference between the control and the stressed animals administered vehicles were maintained at similar level for the remainder of the experiment (see figures below).

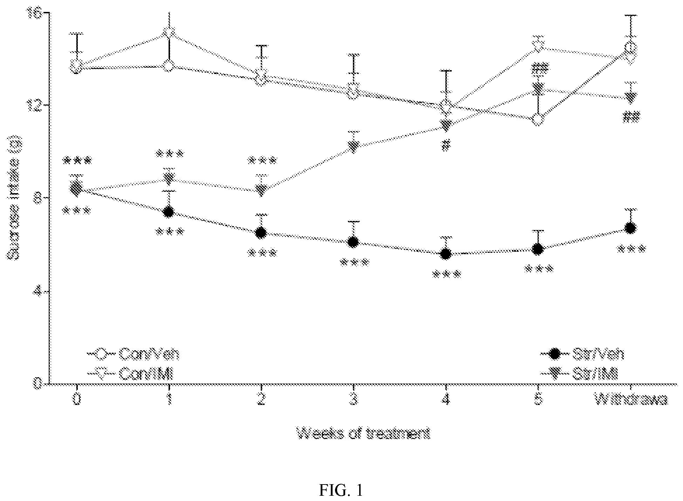

Figure 1 illustrates the effects of chronic treatment with vehicle (1 ml/kg, IP) and imipramine (10 mg/kg, IP) on the consumption of 1% sucrose solution in controls (open symbols) and in animals exposed to chronic mild stress (closed symbols). Treatment commenced following 2 weeks of initial stress. Values are means +/- SEM. As compared to vehicle administration, imipramine had no significant effect on sucrose intakes in control animals [Treatment effect: F(1,84) = 1.379; NS] and gradually increased the sucrose consumption in stressed animals, resulting in a significant Treatment effect [F(1,84) = 66.044; p<0.001] and Treatment x Weeks interaction [F(5.84) = 7.397; p<0.001]. As compared to Week 0 scores, the increases in sucrose intakes in stressed animals administered imipramine reached statistical significance after four weeks of treatment (p=0.038). This effect was enhanced thereafter and was maintained one week after cessation of treatment. *** - p<0.001; relative to vehicle- or drug-treated control groups, # - p<0.0, ## - p<0.01; relative to drug-treated stressed group at Week 0.

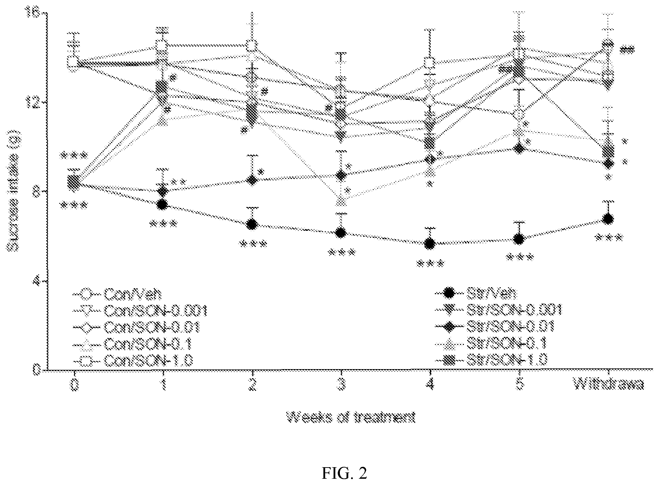

Figure 2 illustrates the effects of chronic treatment with vehicle (1 ml/kg, IP) and SON-117 (0.001, 0.01, 0.1 and 1 mg/kg, IP) on the consumption of 1% sucrose solution in controls (open symbols) and in animals exposed to chronic mild stress (closed symbols). Treatment commenced following 2 weeks of stress. Values are means +/- SEM. As compared to vehicle administration, SON-117 (all doses) had no significant effect on sucrose intake in control animals [Treatment effect: F(4,210) = 1.174; NS] and caused a highly significant Treatment effect [F(4,210) = 20.964; p<0.001] and Treatment x Weeks interaction [F(20,210) = 11.679; p=0.039] in stressed groups. When compared to vehicle injections, all four doses of SON-117 caused significant effects in stressed animals [0.001mg: F(1,84) = 95.974; p<0.001, 0.01mg: F(1,84) = 18.571; p<0.001, 0.1mg: F(1,84) = 33.659; p<0.001, 1mg: F(1,84) = 51.768; p<0.001]. However, when compared to Week 0 scores, this effect reached statistical significance only after administration of the doses of 0.001 and 0.01 mg/kg. These increases of sucrose consumption in stressed animals treated with these doses reached significance after already the first week of administration (p<0.013 and p<0.031, respectively). One week after cessation of treatments the effect of 0.001 mg/kg was maintained but the effect of 1 mg/kg was abolished. * - p<0.05, ** - p<0.01, *** - p<0.001; relative to vehicle- or drug-treated control groups. # - p<0.05, ## - p<0.01; relative to drug-treated stressed groups at Week 0.

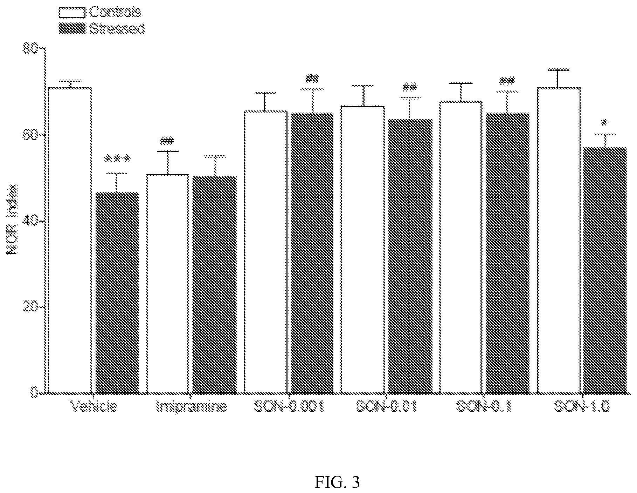

Figure 3 illustrates the effects of chronic treatment with vehicle (1 ml/kg, IP), imipramine (10 mg/kg, IP) and SON-117 (0.001, 0.01, 0.1 and 1 mg/kg, IP) on the behaviour of control (open symbols) and stressed (closed symbols) animals in the Novel Object Recognition test. The test was carried out one week after withdrawal from the treatments. Values are means +/- SEM. The CMS procedure decreased the recognition index, resulting in a significant Group effect [F(1,14) = 22.441; p<0.001]. Imipramine decreased the recognition index in control animals [F(1,14) = 12.101; p<0.01] and was ineffective against the CMS-induced deficit [F(1,14) = 0.287; NS]. SON-117 (all doses) had no significant effect on the recognition index in control animals [F(4,35) = 0.346; NS] and caused a significant Treatment effect [F(4,35) = 2.686, p<0.05] in stressed groups. In stressed animals SON-117, administered at lower doses of 0.001, 0.01 and 0.1 mg/kg, restored the recognition index to the level of the vehicle- or drug-treated control animals, while the highest dose of 1 mg/kg was ineffective against the CMS-induced deficit in the novel object recognition. * - p<0.05, *** - p<0.001; relative to vehicle- or drug-treated control groups. # - p<0.05, ## - p<0.01; relative to vehicle-treated control or stressed groups.

Before the stress procedure was initiated (baseline) the control and to-be-stressed animals had comparable body weights (320 and 316g, respectively) and after initial two weeks of stress (Week 0) the stressed animals were only slightly smaller than the controls (336 and 352 g, respectively). As compared to the vehicle-treated animals, neither imipramine nor SON-117 had any significant effect on the body weights of control [IMI: F1,14) = 3.554; NS, SON-117: F(4,35) = 1.184; NS] and stressed [IMI: F1,14) = 0.879; NS, SON-117: F(4,35) = 2.623; p=0.051; NS].

The results of this study are consistent with previous data showing that the CMS procedure causes a substantial decrease in the consumption of 1% sucrose solution, and that this deficit can be fully reversed by chronic treatment with imipramine. The magnitude of this effect and its onset was comparable to that observed in other CMS studies with this antidepressant. SON-117 appears to be active in the CMS model of depression; the compound had no effects in controls and enhanced the sucrose intakes in stressed animals. The magnitude of action of the most active doses of SON-117 (0.001 and 1.0 mg/kg) was comparable to that of imipramine; by the end of treatment period the sucrose consumption in stressed animals treated with these doses was restored to the pre-stress level. The onset of action of these doses was clearly faster than that of imipramine; the enhancement of sucrose intakes reached statistical significance already following the first week of administration, compared to four weeks required by imipramine. Further, treatment with SON-117 as set forth herein was not associated with the cognitive impairment that is observed with imipramine. The other two doses of 0.01 and 0.1 mg/kg were less effective than imipramine: although the intakes were significantly increased, relative to vehicle-treated stressed animals, they remained suppressed relative to vehicle-treated control animals and to the pre-stress values. Except for the highest doses of 1 mg/kg, the activity of SON-117 in the CMS model was maintained for one week after withdrawal from the treatments. As found in the Novel Object Recognition test, SON-117 can also prevent the stressed animals from the CMS-induced deficit in working memory. Interestingly, this effect was observed only in animals which maintained their enhanced sucrose consumption after withdrawal from the treatments

### Example 5: Pharmacokinetic analysis of SON-117 and SON-117 metabolites.

SON-117 and the SON-117 metabolite M1: 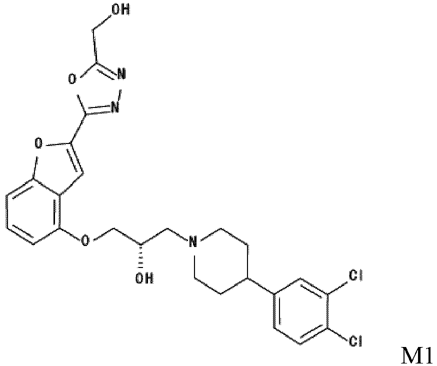 were examined in pharmacokinetic studies.

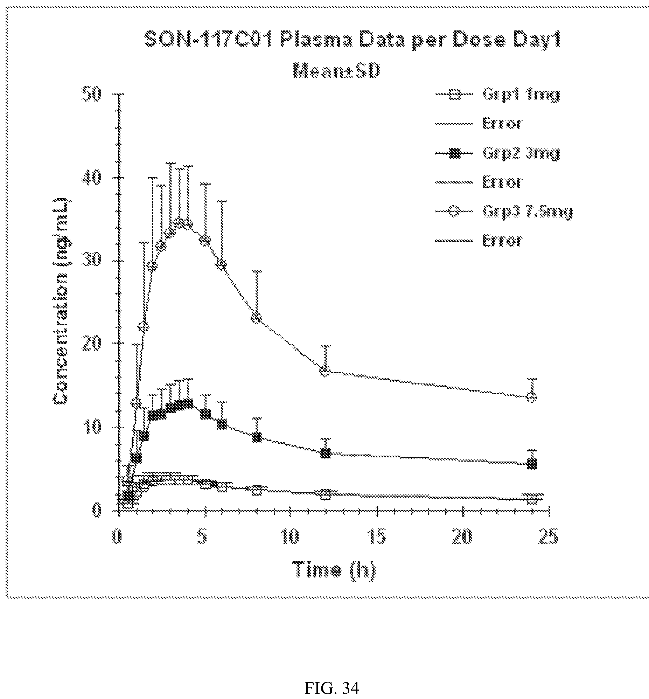

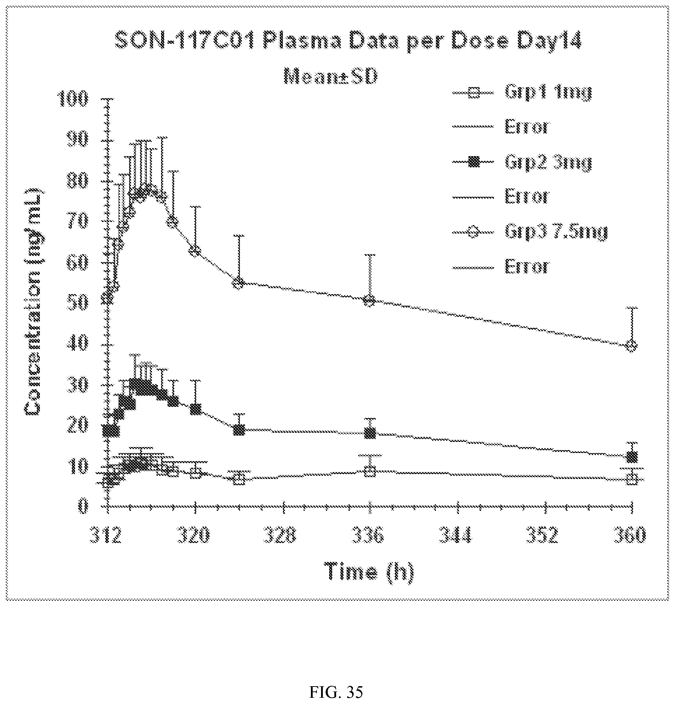

Figure 34 illustrates the plasma concentration of SON-117 as a function of time on day 1 after dosing with SON-117. Figure 35 illustrates the plasma concentration of SON-117 as a function of time on day 14 after dosing with SON-117. These data demonstrate the dose-proportional increase of Cₘₐₓ and AUC for SON-117.

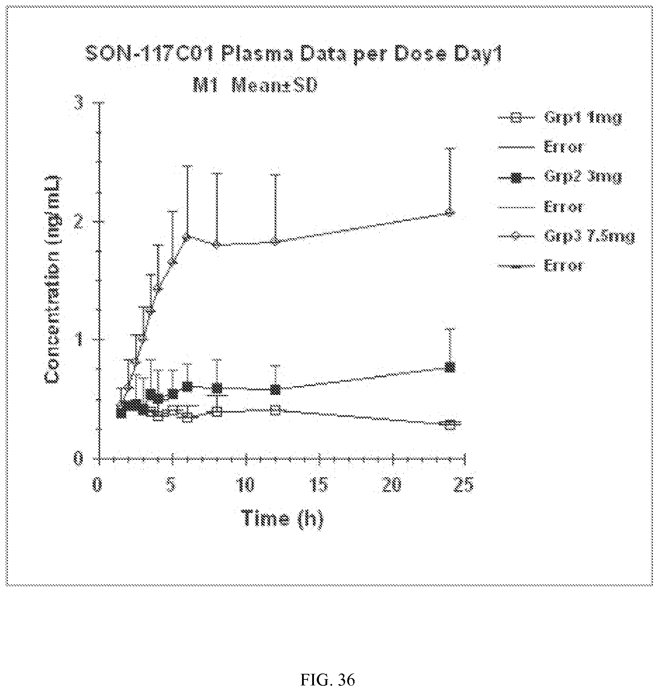

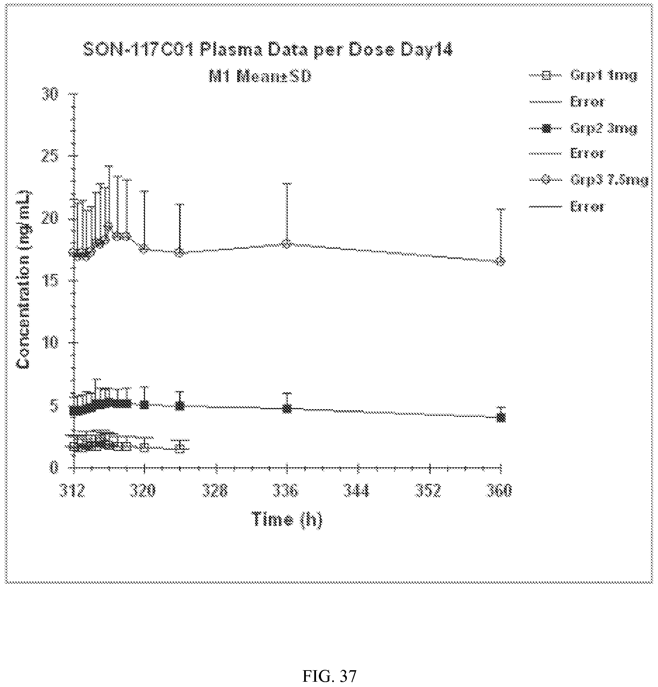

Figure 36 illustrates the plasma concentration of the M1 metabolite of SON-117 as a function of time on day 1 after dosing with SON-117. Figure 37 illustrates the plasma concentration of the M1 metabolite of SON-117 as a function of time on day 14 after dosing with SON-117. These data demonstrate the dose-proportional increase of Cₘₐₓ and AUC for the M1 metabolite of SON-117.

The invention has been described herein by reference to certain preferred embodiments. However, as particular variations thereon will become apparent to those skilled in the art, based on the disclosure set forth herein, the invention is not to be considered as limited thereto.

It is to be understood that at least some of the descriptions of the invention have been simplified to focus on elements that are relevant for a clear understanding of the invention, while eliminating, for purposes of clarity, other elements that those of ordinary skill in the art will appreciate may also comprise a portion of the invention. However, because such elements are well known in the art, and because they do not necessarily facilitate a better understanding of the invention, a description of such elements is not provided herein.

## Claims

1. A composition for use in treating or improving at least one disorder or parameter of sleep in a human subject, the composition comprising the compound of formula (I) or a pharmaceutically acceptable salt, hydrate, or solvate thereof, 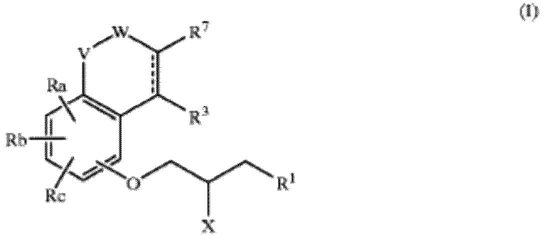
wherein each symbol in the formula means as follows: a bond represented by a solid line and a dotted line shows a double bond or a single bond;
X is a hydrogen atom, a hydroxy group, a C₁-C₈ alkoxy group, an acyloxy group or an oxo group;
R¹ is a group of the following formula 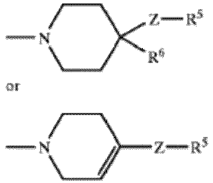
wherein R⁵ is optionally substituted aryl group or optionally substituted aromatic heterocyclic group,
Z is void or --CH₂ --, and
R⁶ is hydrogen atom, hydroxy group, acetamido group, carboxyl group, alkoxycarbonyl group, cyano group or C₁-C₈ alkoxy group;
R³ is a hydrogen atom, a C₁-C₁₈ alkyl group or a halogen atom;
V is --O--;
W is void;
R⁷ is a C₁-C₄ hydroxyalkyl group, an acyl group, an optionally substituted saturated or unsaturated heterocyclic group, an optionally substituted fused heterocyclic group, a C₁ - C₄ alkylsulfonyl group or the formula --Q--R⁹
wherein
Q is --C(=O)--, --C(=S)--, --CH₂ -- or --S(=O)₂--, and
R⁹ is a group of the following formula 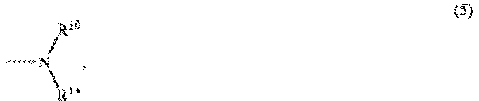 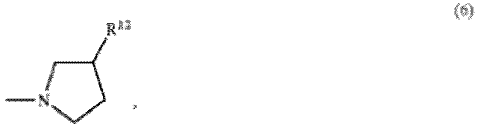 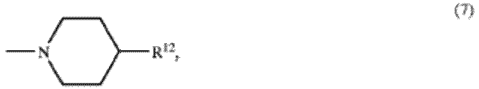 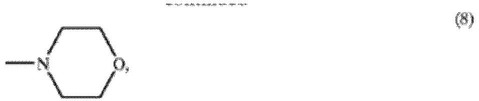 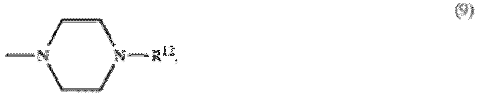 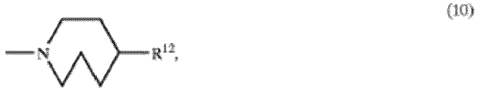 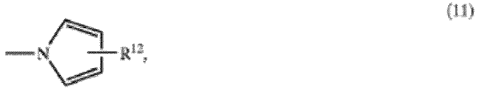 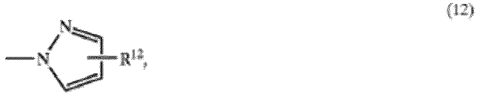 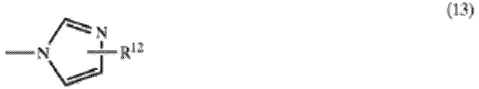
or --NH--NH--R¹⁵
wherein R¹⁰ and R¹¹ are each independently hydrogen atom, C₁-C₁₈ alkyl group, optionally substituted aryl group, optionally substituted aralkyl group or alkoxy group, R¹² is hydrogen atom, optionally substituted aryl group, C₁-C₁₈ alkyl group, C₁-C₈ alkoxy group or acyl group, and R¹⁵ is hydrogen atom, phenyl group, C₁-C₄ alkyl group, C₁-C₂ halogenated alkyl group, halogen atom, C₂-C₄ alkenyl group, C₁-C₄ hydroxyalkyl group, alkoxyalkyl group, alkyloxycarbonyl group, optionally substituted amino group, acetamido group, carboxyl group, acyl group, optionally substituted alkyloxy group, alkylthio group or cyano group;
Ra, Rb and Rc are each independently a hydrogen atom, a C₁-C₁₈ alkyl group, a hydroxy group, a C₁-C₈ alkoxy group, a halogen atom, an acyl group, a nitro group or an amino group;
an optically active compound thereof, a pharmaceutically acceptable salt thereof or a hydrate thereof;
wherein the compound of formula (I) is present in the composition between 0.01 mg/kg and 0.2 mg/kg of the subject's weight, and the subject suffers from depression.

2. The composition for use of claim 1, wherein at least one symptom of depression is also improved or treated in the subject.

3. The composition for use of claim 1, wherein the subject has discontinued treatment with an anti-depressant compound.

4. The composition for use of any preceding claim, wherein the compound of formula I is the compound set forth in formula II:

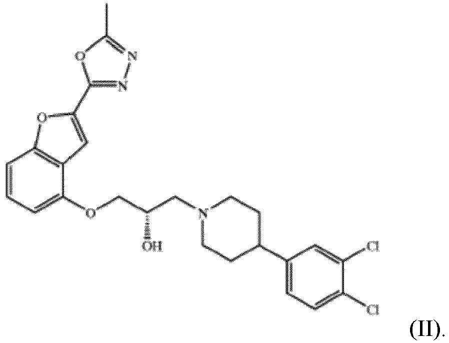

5. The composition for use of any preceding claim, wherein the compound of formula I is in the form of a hydrochloride salt.

6. The composition for use of any one claims 1-3, wherein the compound of formula I is the compound set forth in formula M1:

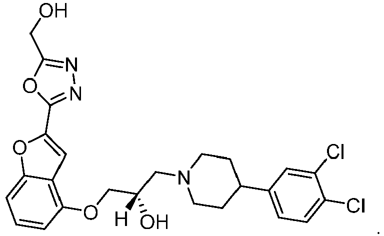

7. The composition for use of any preceding claim, wherein the composition is administered to the human subject once a week, once a day, twice a day, three times a day, four times a day, or five times a day.

## Patentansprüche

1. Zusammensetzung zur Verwendung bei der Behandlung oder Verbesserung von mindestens einer Schlafstörung oder einem Schlafparameter bei einem menschlichen Subjekt, wobei die Zusammensetzung die Verbindung mit der Formel (I) oder ein pharmazeutisch akzeptables Salz, Hydrat oder Solvat davon umfasst, 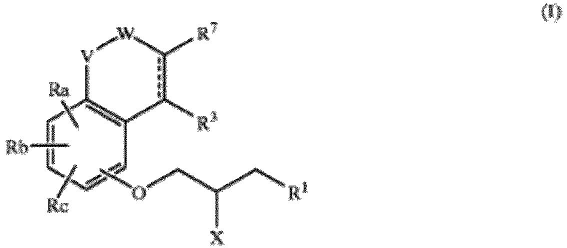 wobei jedes Symbol in der Formel Folgendes bedeutet: eine Bindung, die durch eine durchgehende Linie und eine gepunktete Linie repräsentiert wird, zeigt eine Doppelbindung oder eine Einzelbindung;
X ist ein Wasserstoffatom, eine Hydroxygruppe, eine C₁-C₈-Alkoxygruppe, eine Acyloxygruppe oder eine Oxogruppe;
R¹ ist eine Gruppe mit der folgenden Formel 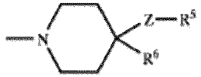 oder 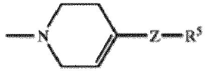
wobei R⁵ eine wahlweise substituierte Arylgruppe oder eine wahlweise substituierte aromatische heterozyklische Gruppe ist,
Z leer oder --CH₂-- ist, und
R⁶ ein Wasserstoffatom, eine Hydroxygruppe, Acetamidogruppe, Carboxylgruppe, Alkoxycarbonylgruppe, Cyanogruppe oder C₁-C₈-Alkoxygruppe ist;
R³ ein Wasserstoffatom, eine C₁-C₁₈-Alkylgruppe oder ein Halogenatom ist;
V --O-- ist;
W leer ist;
R⁷ eine C₁-C₄-Hydroxyalkylgruppe, eine
Acylgruppe, eine wahlweise substituierte, gesättigte oder ungesättigte heterozyklische Gruppe, eine wahlweise substituierte, fusionierte heterozyklische Gruppe, eine C₁-C₄-Alkylsulfonylgruppe oder die Formel --Q--R⁹ ist,
wobei
Q --C(=O)--, --C(=S)--, --CH₂-- oder --S(=O)₂-- ist; und
R⁹ eine Gruppe mit der folgenden Formel 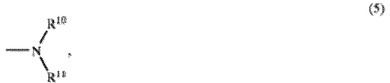 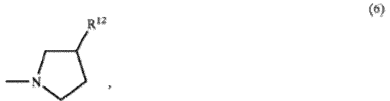 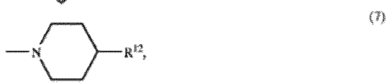 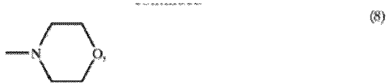 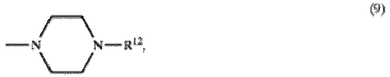 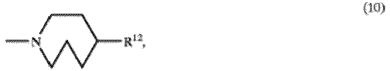 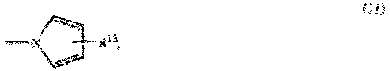 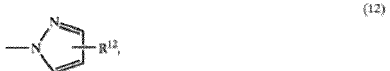 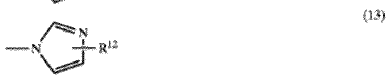
oder --NH--NH--R¹⁵ ist,
wobei R¹⁰ und R¹¹ jeweils unabhängig ein Wasserstoffatom, eine C₁-C₁₈-Alkylgruppe, wahlweise substituierte Arylgruppe, wahlweise substituierte Aralkylgruppe oder Alkoxygruppe sind, R¹² ein Wasserstoffatom, eine wahlweise substituierte Arylgruppe, C₁-C₁₈-Alkylgruppe, C₁-C₈-Alkoxygruppe oder Acylgruppe ist, und R¹⁵ ein Wasserstoffatom, eine Phenylgruppe, C₁-C₄-Alkylgruppe, C₁-C₂-halogenierte Alkylgruppe, ein Halogenatom, eine C₂-C₄-Alkenylgruppe, C₁-C₄-Hydroxyalkylgruppe, Alkoxyalkylgruppe, Alkyloxycarbonylgruppe, wahlweise substituierte Aminogruppe, Acetamidogruppe, Carboxylgruppe, Acylgruppe, wahlweise substituierte Alkyloxygruppe, Alkylthiogruppe oder Cyanogruppe ist;
Ra, Rb und Rc jeweils unabhängig ein Wasserstoffatom, eine C₁-C₁₈-Alkylgruppe, eine Hydroxygruppe, eine C₁-C₈-Alkoxygruppe, ein Halogenatom, eine Acylgruppe, eine Nitrogruppe oder eine Aminogruppe sind;
eine optisch aktive Verbindung davon, ein pharmazeutisch akzeptables Salz davon oder ein Hydrat davon;
wobei die Verbindung mit der Formel (I) in der Zusammensetzung in der Menge zwischen 0,01 mg/kg und 0,2 mg/kg Körpergewicht des Subjekts vorhanden ist und das Subjekt unter Depressionen leidet.

2. Zusammensetzung zur Verwendung nach Anspruch 1, wobei bei dem Subjekt auch mindestens ein Depressionssymptom verbessert oder behandelt wird.

3. Zusammensetzung zur Verwendung nach Anspruch 1, wobei das Subjekt die Behandlung mit einer antidepressiven Verbindung abgebrochen hat.

4. Zusammensetzung zur Verwendung nach einem vorhergehenden Anspruch, wobei die Verbindung mit der Formel I die in Formel II dargelegte Verbindung ist:

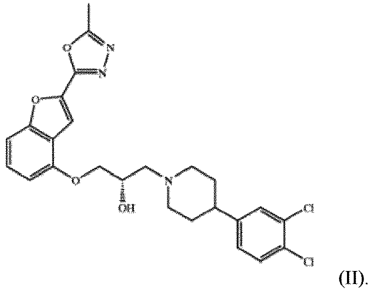

5. Zusammensetzung zur Verwendung nach einem vorhergehenden Anspruch, wobei die Verbindung mit der Formel I in der Form eines Hydrochloridsalzes vorliegt.

6. Zusammensetzung zur Verwendung nach einem der Ansprüche 1-3, wobei die Verbindung mit der Formel I die in Formel M1 dargelegte Verbindung ist:

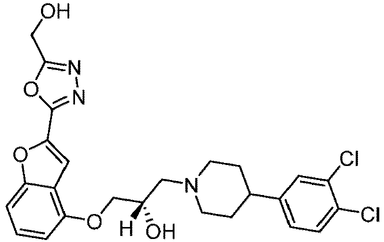

7. Zusammensetzung zur Verwendung nach einem vorhergehenden Anspruch, wobei die Zusammensetzung an das menschliche Subjekt einmal wöchentlich, einmal täglich, zweimal täglich, dreimal täglich, viermal täglich oder fünfmal täglich verabreicht wird.

## Revendications

1. Composition destinée à être utilisée en traitement ou amélioration d'au moins un trouble ou paramètre du sommeil chez un sujet humain, la composition comprenant le composé de formule (I) ou un sel, hydrate ou solvate pharmaceutiquement acceptable de celui-ci, 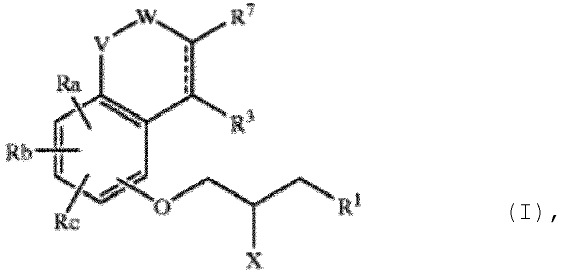 chaque symbole dans la formule signifiant ce qui suit : une liaison représentée par un trait continu et un trait pointillé indique une double liaison ou une liaison simple ;
X est un atome d'hydrogène, un groupe hydroxy, un groupe alcoxy en C₁-C₈, un groupe acyloxy ou un groupe oxo ;
R¹ est un groupe de formule suivante 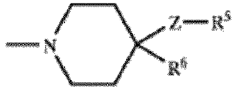 ou 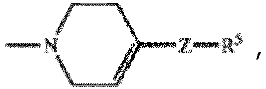
R⁵ étant un groupe aryle éventuellement substitué ou un groupe hétérocyclique aromatique éventuellement substitué,
Z étant absent ou -CH₂- et
R⁶ étant un atome d'hydrogène, un groupe hydroxy, un groupe acétamido, un groupe carboxyle, un groupe alcoxycarbonyle, un groupe cyano ou un groupe alcoxy en C₁-C₈ ;
R³ est un atome d'hydrogène, un groupe alkyle en C₁-C₁₈ ou un atome d'halogène ;
V est -O- ;
W est absent ;
R⁷ est un groupe hydroxyalkyle en C₁-C₄, un groupe acyle, un groupe hétérocyclique saturé ou insaturé éventuellement substitué, un groupe hétérocyclique condensé éventuellement substitué, un groupe alkylsulfonyle en C₁-C₄ ou la formule - Q-R⁹,
Q étant -C(=O)-, -C(=S)-, -CH₂- ou -S(=O)₂- et
R⁹ étant un groupe de formule suivante 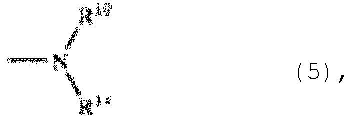 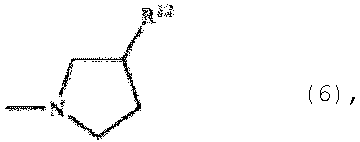 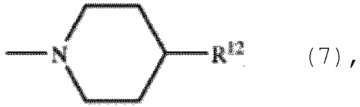 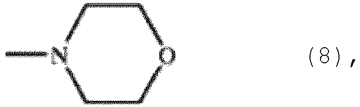 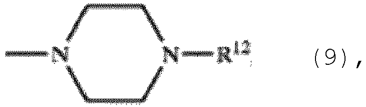 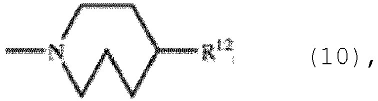 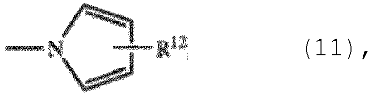 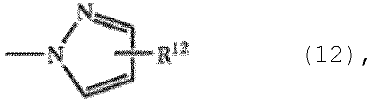 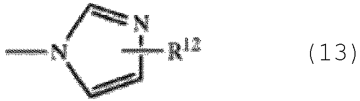 ou -NH-NH-R¹⁵,
R¹⁰ et R¹¹ étant chacun indépendamment un atome d'hydrogène, un groupe alkyle en C₁-C₁₈, un groupe aryle éventuellement substitué, un groupe aralkyle éventuellement substitué ou un groupe alcoxy, R¹² étant un atome d'hydrogène, un groupe aryle éventuellement substitué, un groupe alkyle en C₁-C₁₈, un groupe alcoxy en C₁-C₈ ou un groupe acyle et R¹⁵ étant un atome d'hydrogène, un groupe phényle, un groupe alkyle en C₁-C₄, un groupe alkyle halogéné en C₁-C₂, un atome d'halogène, un groupe alcényle en C₂-C₄, un groupe hydroxyalkyle en C₁-C₄, un groupe alcoxyalkyle, un groupe alkyloxycarbonyle, un groupe amino éventuellement substitué, un groupe acétamido, un groupe carboxyle, un groupe acyle, un groupe alkyloxy éventuellement substitué, un groupe alkylthio ou un groupe cyano ;
Ra, Rb et Rc sont chacun indépendamment un atome d'hydrogène, un groupe alkyle en C₁-C₁₈, un groupe hydroxy, un groupe alcoxy en C₁-C₈, un atome d'halogène, un groupe acyle, un groupe nitro ou un groupe amino ;
un composé optiquement actif de celui-ci, un sel pharmaceutiquement acceptable de celui-ci ou un hydrate de celui-ci ;
le composé de formule (I) étant présent dans la composition entre 0,01 mg/kg et 0,2 mg/kg du poids du sujet et le sujet souffrant de dépression.

2. Composition destinée à être utilisée selon la revendication 1, au moins un symptôme de dépression étant également amélioré ou traité chez le sujet.

3. Composition destinée à être utilisée selon la revendication 1, le sujet ayant un traitement par un composé antidépresseur interrompu.

4. Composition destinée à être utilisée selon une quelconque revendication précédente, le composé de formule I étant le composé présenté dans la formule II :

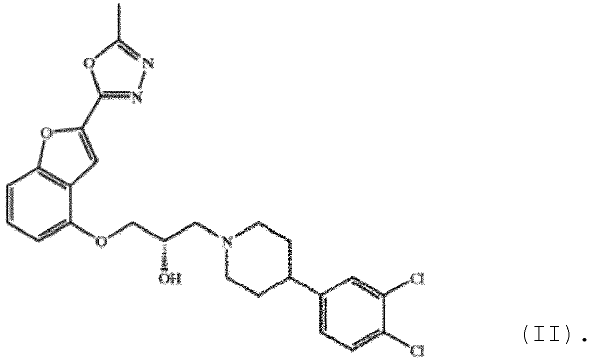

5. Composition destinée à être utilisée selon une quelconque revendication précédente, le composé de formule I étant sous la forme d'un sel de type chlorhydrate.

6. Composition destinée à être utilisée selon l'une quelconque des revendications 1-3, le composé de formule I étant le composé présenté dans la formule M1 :

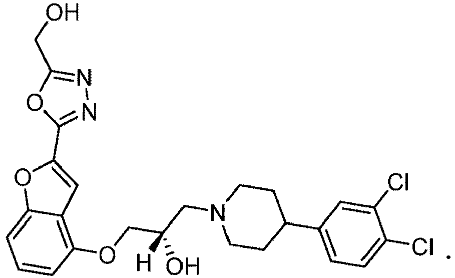

7. Composition destinée à être utilisée selon une quelconque revendication précédente, la composition étant administrée au sujet humain une fois par semaine, une fois par jour, deux fois par jour, trois fois par jour, quatre fois par jour ou cinq fois par jour.
